# EUROPEAN PATENT APPLICATION

(11) **EP 4 020 491 A1**
(43) Date of publication of application: **29.06.2022**
(21) Application number: 20857343.6
(22) Date of filing: 16.07.2020
(51) Int. Cl.: G16H 20/30

(54) **FITNESS-ASSISTED METHOD AND ELECTRONIC APPARATUS**

(30) Priority: 30.08.2019 CN 201910817978
(71) Applicant: Huawei Technologies Co., Ltd., Longgang Shenzhen, Guangdong 518129 (CN)
(72) Inventor: JIANG, Yonghang, Shenzhen, Guangdong 518129 (CN)
(74) Representative: Gill Jennings & Every LLP
(86) International application number: PCT/CN2020/102394
(87) International publication number: WO 2021/036568

(57) **Abstract**

A method for assisting fitness in the field of artificial intelligence is provided, including: An electronic device obtains a user movement (S601); the electronic device determines from the user movement, a candidate movement in which a motion track of a first body part in the user movement meets a first preset condition (S602); the electronic device determines a movement change amplitude of a second body part in the candidate movement (S603); and the electronic device determines, based on the movement change amplitude, to output guidance information (S604). A candidate movement in which a motion track of a body part meets the first preset condition is determined, and it can be accurately determined, based on a movement change amplitude of a body part in the candidate movement, that the user performs a fitness movement. The guidance information is output when it is determined that the user performs the fitness movement, so that accuracy of the output guidance information can be improved, and user experience can be improved.

## Description

This application claims priority to Chinese Patent Application No. 201910817978.X, filed with the China National Intellectual Property Administration on August 30, 2019 and entitled "METHOD FOR ASSISTING FITNESS AND ELECTRONIC APPARATUS", which is incorporated herein by reference in its entirety.

### TECHNICAL FIELD

This application relates to the field of artificial intelligence (artificial intelligence, AI), and in particular, to the field of image processing, and in particular, to a method for assisting fitness and an apparatus.

### BACKGROUND

Fitness needs professional guidance. Without professional guidance, not only a training effect is difficult to achieve, but also serious sports injuries may be produced. The price of guidance from a professional coach is high, and it is difficult to meet needs of fitness enthusiasts. A fitness movement of a user is identified through image recognition, completion quality of the user is evaluated based on a key indicator of the fitness movement, and a wrong movement is pointed out and an improvement method is provided. This provides guidance for the user, so that the user can exercise scientifically.

In a fitness process, the user may perform other movements unrelated to fitness. Accordingly, a relatively large calculation amount is required for evaluating these movements, and user experience is poor. A movement template of the user is compared with a standard template, to determine, based on a similarity or difference, whether the user performs a fitness movement. In this case, accuracy of a determining result is relatively low.

### SUMMARY

This application provides a method for assisting fitness and an electronic apparatus, to accurately recognize a fitness movement, and improve user experience.

According to a first aspect, a method for assisting fitness is provided, including: An electronic device obtains a user movement; the electronic device determines, from the user movement, a candidate movement in which a motion track of a first body part in the user movement meets a first preset condition; the electronic device determines a movement change amplitude of a second body part in the candidate movement; and the electronic device determines, based on the movement change amplitude, to output guidance information.

A candidate movement in which a motion track of a body part meets the first preset condition is determined, and it can be accurately determined, based on a movement change amplitude of a body part in the candidate movement, to output the guidance information. This can improve user experience.

With reference to the first aspect, in some possible implementations, the method further includes: The electronic device obtains input information; and the electronic device determines the first preset condition based on the input information.

The first preset condition is determined based on the obtained input information, to reduce a calculation amount, and improve accuracy of the output guidance information.

With reference to the first aspect, in some possible implementations, the electronic device determines first evaluation information corresponding to first location information, where the first location information includes at least one of the movement change amplitude of the second body part, a movement start location of the second body part, a movement end location of the second body part, and a motion track of the second body part, and the movement start location of the second body part and the movement end location of the second body part are determined based on the motion track; and the electronic device outputs the guidance information based on the first evaluation information.

The first evaluation information corresponding to the first location information of the first body part of the user in a user movement video is determined based on a correspondence between location information of the first body part and evaluation information, to provide guidance on a fitness movement of the user. When only the location information of the first body part is available, guidance on the user movement can be provided, to improve applicability.

With reference to the first aspect, in some possible implementations, the method further includes: The electronic device determines second evaluation information corresponding to second location information of the user, where the second location information includes at least one of a movement change amplitude of a third body part, a movement start location of the third body part, a movement end location of the third body part, and a motion track of the third body part, and the movement start location of the third body part and the movement end location of the third body part are determined based on the motion track of the first body part. That the electronic device outputs the guidance information based on the first evaluation information includes: The electronic device outputs the guidance information based on the second evaluation information and the first evaluation information.

When the user movement further includes the second body part, the second evaluation information corresponding to the second location information of the second body part of the user in the user movement video may be determined based on a correspondence between location information of the second body part and evaluation information, to provide more comprehensive and detailed guidance on the fitness movement of the user.

With reference to the first aspect, in some possible implementations, the electronic device recognizes joints in the user movement, to determine the first body part and the second body part in the user movement.

The electronic device determines the first body part by recognizing skeleton joints, so that a calculation amount can be reduced.

With reference to the first aspect, in some possible implementations, the method further includes: The electronic device obtains input information, where the input information is used to indicate a fitness movement; and the electronic device determines the first body part corresponding to the fitness movement.

The electronic device determines the first body part based on the input information indicating the fitness movement, so that a calculation amount can be reduced.

With reference to the first aspect, in some possible implementations, that the electronic device determines, based on the movement change amplitude, to output guidance information includes: The electronic device determines, based on the movement change amplitude and the movement start location of the second body part in the candidate movement, to output the guidance information; or the electronic device determines, based on the movement change amplitude and the movement end location of the second body part in the candidate movement, to output the guidance information; or the electronic device determines, based on the movement change amplitude, and the movement start location and the movement end location of the second body part in the candidate movement, to output the guidance information, where the movement start location of the second body part and the movement end location of the second body part are determined based on the motion track of the first body part.

The electronic device determines, based on the movement change amplitude and one or more of the movement start location and the movement end location of the second body part in the candidate movement, to output the guidance information. That is, the user movement is determined by using more location information of the second body part, to improve recognition accuracy of the fitness movement. More accurate guidance information can be output, and user experience is improved.

According to a second aspect, an apparatus for assisting fitness is provided, including: an obtaining module, configured to obtain a user movement; and a determining module, configured to determine, from the user movement, a candidate movement in which a motion track of a first body part in the user movement meets a first preset condition. The determining module is further configured to determine a movement change amplitude of a second body part in the candidate movement; and the determining module is further configured to determine, based on the movement change amplitude, to output guidance information.

With reference to the second aspect, the apparatus further includes a judgment module, configured to determine that the movement change amplitude meets a second preset condition, and the determining module is configured to determine to output the guidance information.

With reference to the second aspect, in some possible implementations, the obtaining module is further configured to obtain input information, and the determining module is further configured to determine the first preset condition corresponding to the input information.

With reference to the second aspect, in some possible implementations, the determining module is further configured to determine first evaluation information corresponding to first location information, where the first location information includes at least one of the movement change amplitude of the second body part, a movement start location of the second body part, a movement end location of the second body part, and a motion track of the second body part, and the movement start location of the second body part and the movement end location of the second body part are determined based on the motion track of the first body part. The apparatus further includes an output module, configured to output the guidance information based on the first evaluation information.

With reference to the second aspect, in some possible implementations, the determining module is further configured to determine second evaluation information corresponding to second location information of a user, where the second location information includes at least one of a movement change amplitude of a third body part, a movement start location of the third body part, a movement end location of the third body part, and a motion track of the third body part, and the movement start location of the third body part and the movement end location of the third body part are determined based on the motion track of the first body part. The output module is further configured to output the guidance information based on the second evaluation information and the first evaluation information.

With reference to the second aspect, in some possible implementations, the apparatus further includes a recognition module, configured to recognize joints in the user movement, to determine the first body part and the second body part in the user movement.

With reference to the second aspect, in some possible implementations, the determining module is further configured to determine, based on the movement change amplitude and the movement start location of the second body part in the candidate movement, to output the guidance information; or determine, based on the movement change amplitude and the movement end location of the second body part in the candidate movement, to output the guidance information; or determine, based on the movement change amplitude, and the movement start location and the movement end location of the second body part in the candidate movement, to output the guidance information, where the movement start location of the second body part and the movement end location of the second body part are determined based on the motion track of the first body part.

According to a third aspect, an apparatus for assisting fitness is provided, including a processor and a communications interface. The communications interface is configured to obtain a user movement. The processor is configured to determine, from the user movement, a candidate movement in which a motion track of a first body part in the user movement meets a first preset condition; determine a movement change amplitude of a second body part in the candidate movement; and determine, based on the movement change amplitude, to output guidance information.

With reference to the third aspect, in some possible implementations, the processor is configured to determine that the movement change amplitude meets a second preset condition, and determine to output the guidance information.

With reference to the third aspect, in some possible implementations, the communications interface is further configured to obtain input information, and the processor is further configured to determine the first preset condition corresponding to the input information.

With reference to the third aspect, in some possible implementations, the processor is configured to determine first evaluation information corresponding to first location information, where the first location information includes at least one of the movement change amplitude of the first body part, a movement start location of the first body part, a movement end location of the first body part, and a motion track of the first body part, and the movement start location of the first body part and the movement end location of the second body part are determined based on the motion track of the first body part; and determine the guidance information based on the first evaluation information.

With reference to the third aspect, in some possible implementations, the processor is configured to determine second evaluation information corresponding to second location information of the user, where the second location information includes at least one of a movement change amplitude of a third body part, a movement start location of the third body part, a movement end location of the third body part, and a motion track of the third body part, and the movement start location of the third body part and the movement end location of the third body part are determined based on the motion track of the first body part; and determine the guidance information based on the second evaluation information and the first evaluation information.

With reference to the third aspect, in some possible implementations, the processor is further configured to recognize joints in the user movement, to determine the first body part and the second body part in the user movement.

With reference to the third aspect, in some possible implementations, the processor is further configured to determine, based on the movement change amplitude and the movement start location of the second body part in the candidate movement, to output the guidance information; or determine, based on the movement change amplitude and the movement end location of the second body part in the candidate movement, to output the guidance information; or determine, based on the movement change amplitude, and the movement start location and the movement end location of the second body part in the candidate movement, to output the guidance information, where the movement start location of the second body part and the movement end location of the second body part are determined based on the motion track of the first body part.

According to a fourth aspect, a computer storage medium is provided. When the computer instructions are run on an electronic device, the electronic device is enabled to perform the method according to the first aspect.

According to a fifth aspect, a chip system is provided. The chip system includes at least one processor, and when program instructions are executed in the at least one processor, the chip system is enabled to perform the method according to the first aspect.

### BRIEF DESCRIPTION OF DRAWINGS

FIG. 1 is a schematic diagram of a hardware structure of an electronic device;
FIG. 2 is a schematic diagram of a software structure of an electronic device;
FIG. 3 is a schematic flowchart of a method for assisting fitness according to an embodiment of this application;
FIG. 4 is a schematic flowchart of a method for assisting fitness according to another embodiment of this application;
FIG. 5(a) to FIG. 5(c) are schematic flowcharts of user interfaces for assisting fitness according to an embodiment of this application;
FIG. 6 is a schematic flowchart of a user interface for assisting fitness according to another embodiment of this application;
FIG. 7 is a schematic flowchart of a method for assisting fitness according to still another embodiment of this application;
FIG. 8(a) and FIG. 8(b) are schematic diagrams of a squat movement;
FIG. 9 is a schematic diagram of a structure of an electronic apparatus according to an embodiment of this application; and
FIG. 10 is a schematic diagram of a structure of an electronic apparatus according to another embodiment of this application.

### DESCRIPTION OF EMBODIMENTS

The following describes technical solutions of this application with reference to the accompanying drawings.

The group of fitness enthusiasts is growing gradually, and fitness needs professional guidance. Without professional guidance, not only a training effect is difficult to achieve, but also serious sports injuries may be produced. Professional private instructors are not only few in quantity, but also high in price, and it is difficult to meet needs of all the fitness enthusiasts.

A quantity of times that a user completes movements may be recorded through image recognition of fitness movements of the user, and completion quality of the user is evaluated based on a key indicator of the fitness movement, and a wrong movement is pointed out and an improvement method is provided, to provide scientific guidance for the user. Joint information of the user's body may be collected by using collected images of the fitness movement of the user, and a posture of the user is compared with a posture of a standard movement based on the joint information of the user and joint information of the standard movement, to determine a difference between the user movement and the standard movement, so as to provide feedback and guidance for the user.

In a fitness process, the user may perform some movements unrelated to fitness, for example, taking something, answering a phone, and walking. When the user does not perform a fitness movement, these unrelated movements are considered as non-standard fitness movements. Evaluation and guidance are still provided, and this leads to poor user experience.

When the similarity between the user movement and the standard movement is lower than a preset value, evaluation and guidance on the user movement may not be provided.

For different movements, different body parts have different effects on degrees of completion of the movements. Different weights may be set for different body parts, and only a posture of a body part related to the movement is evaluated. For example, there are three most important evaluation indicators for a squat movement, namely, a shin angle, a thigh angle, and a torso angle. Angles of other body parts hardly affect accuracy of the squat movement. A weight of impact of a posture of another body part on a degree of completion of the movement is set to 0, and only weights of the shin, the thigh, and the torso are greater than 0. A difference between a posture of another body part and the standard movement does not affect evaluation of the squat movement, and an excessive low similarity due to a large difference between the movement of another body part and the standard movement can be avoided, so that correct evaluation and guidance can be triggered.

However, in practice, a movement of a fitness beginner, especially a complex movement that requires cooperation of different body parts, may be not standard, and the movement performed by the beginner may not meet a similarity requirement. Consequently, the movement is determined as an unrelated movement, effective guidance cannot be provided, and user experience is poor.

Still in the example of the squat movement, when a user movement is not standard, for example, when the torso and the shin lean forward excessively, an overall similarity is still relatively low, and may be lower than a preset threshold. Consequently, the user movement is determined as an unrelated movement, and evaluation and guidance are not triggered.

Therefore, in a fitness scenario, a similarity is determined by statically comparing a gesture of a user movement with a gesture of a standard movement, and it cannot be accurately recognized whether the user performs a fitness movement.

The user movement may be a continuous movement within a period of time. When the user movement is compared with the standard movement, the user movement at a time point or in a period of time is compared with the static standard movement. To determine the similarity between the gesture of the user movement and the gesture of the standard movement, a dynamic time warping (dynamic time warping, DTW) technology may usually be used to determine a time window, and a user movement in the time window is compared with the standard movement. Ideally, start time and end time of the user movement are determined, and are respectively used as a start point and an end point of the time window, and the user movement in the time window is compared with the standard movement. In practice, durations of different movements of different users are different, and it is difficult to accurately determine start time and end time of a user movement. That is, it is difficult to determine a time window. An excessively large window causes relatively high calculation overheads, and a similarity obtained by calculation is relatively low. In an excessively small window, it may be impossible to recognize whether the user performs a fitness movement. In addition, in this method, a similarity is determined by comparing a gesture of a user movement with a gesture of a standard movement, and it cannot be accurately recognized whether the user performs a fitness movement.

To resolve the foregoing problem, this application provides a method for assisting fitness, to recognize whether the user performs a fitness movement. The method may be performed in the electronic device.

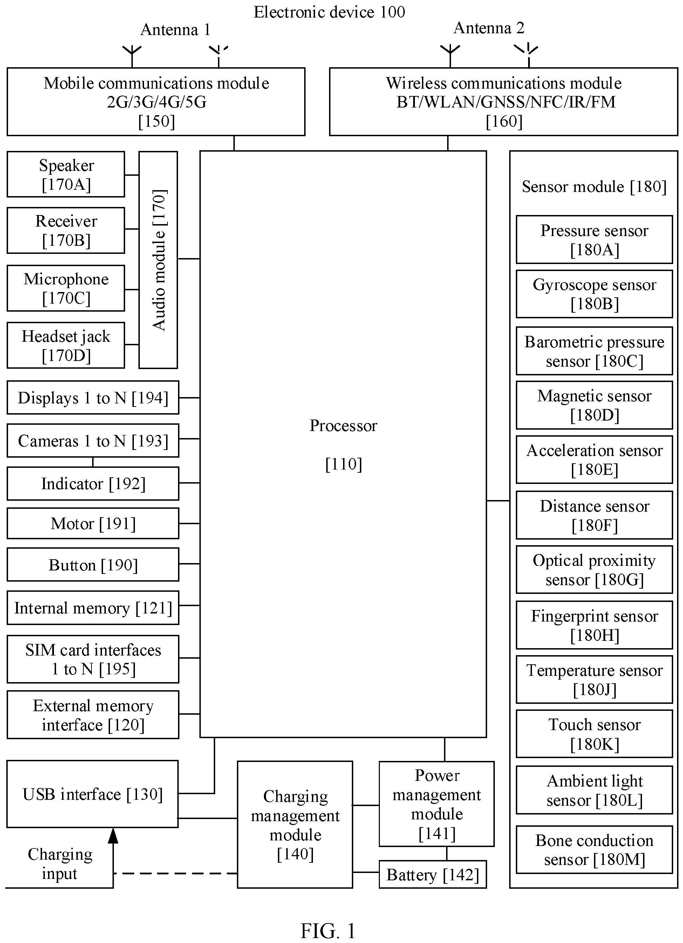

For example, FIG. 1 is a schematic structural diagram of an electronic device 100. The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communications module 150, a wireless communications module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in the embodiments of this application does not constitute a specific limitation on the electronic device 100. In some other embodiments of this application, the electronic device 100 may include more or fewer components than those shown in the figure, or some components may be combined, or some components may be split, or there may be a different component layout. The components shown in the figure may be implemented by hardware, software, or a combination of software and hardware.

For example, the electronic device in the embodiments of this application may include the processor 110, the audio module 170, the speaker 170A, a Bluetooth module in the wireless communications module 160, the display 194, the camera 193, the internal memory 121, and the like.

The processor 110 may include one or more processing units. For example, the processor 110 may include at least one of an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, and a neural-network processing unit (neural-network processing unit, NPU). Different processing units may be independent components, or may be integrated into one or more processors.

The controller may be a nerve center and a command center of the electronic device 100. The controller may generate an operation control signal based on an instruction operation code and a time sequence signal, to control to read instructions and execute instructions.

A memory may be further disposed in the processor 110, and is configured to store an instruction and data. In some embodiments, the memory in the processor 110 is a cache. The memory may store instructions or data that has just been used or is cyclically used by the processor 110. If the processor 110 needs to use the instructions or the data again, the processor 110 may directly invoke the instructions or the data from the memory, to avoid repeated access. This reduces a waiting time of the processor 110, and improves system efficiency.

In some embodiments, the processor 110 may include one or more interfaces. The interface may include at least one of an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and the like.

The I2C interface is a two-way synchronization serial bus, and includes a serial data line (serial data line, SDA) and a serial clock line (serial clock line, SCL). In some embodiments, the processor 110 may include a plurality of groups of I2C buses. The processor 110 may be separately coupled to the touch sensor 180K, a charger, a flash light, the camera 193, and the like through different I2C bus interfaces. For example, the processor 110 may be coupled to the touch sensor 180K through the I2C interface, so that the processor 110 communicates with the touch sensor 180K through the I2C bus interface, to implement a touch function of the electronic device 100.

The 12S interface may be configured to perform audio communication. In some embodiments, the processor 110 may include a plurality of groups of 12S buses. The processor 110 may be coupled to the audio module 170 through an I2S bus, to implement communication between the processor 110 and the audio module 170. In some embodiments, the audio module 170 may transmit an audio signal to the wireless communications module 160 through the I2S interface, to implement a function of answering a call by using a Bluetooth headset.

The PCM interface may also be configured to perform audio communication, and sample, quantize, and code an analog signal. In some embodiments, the audio module 170 may be coupled to the wireless communications module 160 through the PCM bus interface. In some embodiments, the audio module 170 may also transmit an audio signal to the wireless communications module 160 through the PCM interface, to implement a function of answering a call by using the Bluetooth headset. Both the I2S interface and the PCM interface may be configured to perform audio communication.

The UART interface is a universal serial data bus, and is configured to perform asynchronous communication. The bus may be a two-way communications bus, and converts to-be-transmitted data between serial communication and parallel communication. In some embodiments, the UART interface is usually configured to connect the processor 110 to the wireless communications module 160. For example, the processor 110 communicates with a Bluetooth module in the wireless communications module 160 through the UART interface, to implement a Bluetooth function. In some embodiments, the audio module 170 may transmit an audio signal to the wireless communications module 160 through the UART interface, to implement a function of playing music by using the Bluetooth headset.

The MIPI interface may be configured to connect the processor 110 to a peripheral component such as the display 194 or the camera 193. The MIPI interface includes a camera serial interface (camera serial interface, CSI), a display serial interface (display serial interface, DSI), and the like. In some embodiments, the processor 110 communicates with the camera 193 through the CSI interface, to implement a photographing function of the electronic device 100. The processor 110 communicates with the display 194 through the DSI interface, to implement a display function of the electronic device 100.

The GPIO interface may be configured by using software. The GPIO interface may be configured as a control signal or a data signal. In some embodiments, the GPIO interface may be configured to connect the processor 110 to the camera 193, the display 194, the wireless communications module 160, the audio module 170, the sensor module 180, and the like. The GPIO interface may alternatively be configured as the I2C interface, the I2S interface, the UART interface, the MIPI interface, or the like.

The USB interface 130 is an interface that complies with a USB standard specification, and may be specifically a mini USB interface, a micro USB interface, a USB Type-C interface, or the like. The USB interface 130 may be configured to connect to a charger to charge the electronic device 100, or may be configured to transmit data between the electronic device 100 and a peripheral device, or may be used to connect to a headset for playing audio by using the headset. Alternatively, the interface may be configured to connect to another electronic device such as an AR device.

It may be understood that an interface connection relationship between modules illustrated in this embodiment of this application is merely an example for description, and does not constitute a limitation on the structure of the electronic device 100. In some other embodiments of this application, the electronic device 100 may alternatively use an interface connection mode different from that in the foregoing embodiment, or use a combination of a plurality of interface connection modes.

The charging management module 140 is configured to receive a charging input from the charger. The charger may be a wireless charger or a wired charger. In some embodiments in which wired charging is used, the charging management module 140 may receive a charging input from the wired charger through the USB interface 130. In some embodiments in which wireless charging is used, the charging management module 140 may receive a wireless charging input through a wireless charging coil of the electronic device 100. The charging management module 140 may further supply power to the electronic device by using the power management module 141 while charging the battery 142.

The power management module 141 is configured to connect the battery 142 and the charging management module 140 to the processor 110. The power management module 141 receives an input from the battery 142 and/or the charging management module 140, and supplies power to the processor 110, the internal memory 121, an external memory, the display 194, the camera 193, the wireless communications module 160, and the like. The power management module 141 may be further configured to monitor parameters such as a battery capacity, a quantity of battery cycles, and a battery health status (electric leakage or impedance). In some other embodiments, the power management module 141 may alternatively be disposed in the processor 110. In some other embodiments, the power management module 141 and the charging management module 140 may alternatively be disposed in a same device.

A wireless communications function of the electronic device 100 may be implemented through the antenna 1, the antenna 2, the mobile communications module 150, the wireless communications module 160, the modem processor, the baseband processor, and the like.

The antenna 1 and the antenna 2 each are configured to transmit and receive an electromagnetic wave signal. Each antenna in the electronic device 100 may be configured to cover one or more communication frequency bands. Different antennas may further be multiplexed to improve antenna utilization. For example, the antenna 1 may be multiplexed as a diversity antenna in a wireless local area network. In some other embodiments, an antenna may be used in combination with a tuning switch.

The mobile communications module 150 may provide a solution that is for wireless communication including 2G/3G/4G/5G and the like and that is applied to the electronic device 100. The mobile communications module 150 may include at least one filter, a switch, a power amplifier, a low noise amplifier (low noise amplifier, LNA), and the like. The mobile communications module 150 may receive an electromagnetic wave through the antenna 1, perform processing such as filtering and amplification on the received electromagnetic wave, and transmit the processed electromagnetic wave to the modem processor for demodulation. The mobile communications module 150 may further amplify a signal modulated by the modem processor, and convert the signal into an electromagnetic wave for radiation through the antenna 1. In some embodiments, at least some function modules of the mobile communications module 150 may be disposed in the processor 110. In some embodiments, at least some function modules in the mobile communications module 150 and at least some modules in the processor 110 may be disposed in a same device.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor, and then transmitted to the application processor. The application processor outputs a sound signal through an audio device (which is not limited to the speaker 170A, the receiver 170B, or the like), or displays an image or a video by using the display 194. In some embodiments, the modem processor may be an independent component. In some other embodiments, the modem processor may be independent of the processor 110, and is disposed in a same device as the mobile communications module 150 or another function module.

The wireless communications module 160 may provide a wireless communications solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like and that is applied to the electronic device 100. The wireless communications module 160 may be one or more components integrated into at least one communications processing module. The wireless communications module 160 receives an electromagnetic wave through the antenna 2, performs frequency modulation and filtering processing on an electromagnetic wave signal, and sends a processed signal to the processor 110. The wireless communications module 160 may further receive a to-be-sent signal from the processor 110, perform frequency modulation and amplification on the signal, and convert the signal into an electromagnetic wave for radiation through the antenna 2.

In some embodiments, in the electronic device 100, the antenna 1 is coupled to the mobile communications module 150, and the antenna 2 is coupled to the wireless communications module 160, so that the electronic device 100 can communicate with a network and another device by using a wireless communications technology. The wireless communications technology may include at least one of a global system for mobile communications (global system for mobile communications, GSM), a general packet radio service (general packet radio service, GPRS), code division multiple access (code division multiple access, CDMA), wideband code division multiple access (wideband code division multiple access, WCDMA), time division code division multiple access (time-division code division multiple access, TD-SCDMA), Long Term Evolution (Long Term Evolution, LTE), BT, a GNSS, a WLAN, NFC, FM, an IR technology, and the like. The GNSS may include a global positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and a satellite based augmentation system (satellite based augmentation system, SBAS).

The electronic device 100 implements a display function by using the GPU, the display 194, the application processor, and the like. The GPU is a microprocessor for image processing, and is connected to the display 194 and the application processor. The GPU is configured to perform mathematical and geometric calculation, and is used for graphics rendering. The processor 110 may include one or more GPUs that execute a program instruction to generate or change display information.

The display 194 is configured to display an image, a video, and the like. The display 194 includes a display panel. The display panel may use a liquid crystal display (liquid crystal display, LCD), an organic light-emitting diode (organic light-emitting diode, OLED), an active-matrix organic light emitting diode (active-matrix organic light emitting diode, AMOLED), a flex light-emitting diode (flex light-emitting diode, FLED), a mini-LED, a micro-LED, a micro-OLED, a quantum dot light emitting diode (quantum dot light emitting diode, QLED), or the like. In some embodiments, the electronic device 100 may include one or N displays 194, where N is a positive integer greater than 1.

The electronic device 100 may implement a photographing function by using the ISP, the camera 193, the video codec, the GPU, the display 194, the application processor, and the like.

The ISP is configured to process data fed back by the camera 193. For example, during photographing, a shutter is pressed, light is transmitted to a photosensitive element of the camera through a lens, an optical signal is converted into an electrical signal, and the photosensitive element of the camera transmits the electrical signal to the ISP for processing, to convert the electrical signal into a visible image. The ISP may further perform algorithm optimization on noise, brightness, and complexion of the image. The ISP may further optimize parameters such as exposure and a color temperature of a photographing scenario. In some embodiments, the ISP may be disposed in the camera 193.

The camera 193 is configured to capture a static image or a video. An optical image of an object is generated by using the lens, and is projected onto a photosensitive element. The photosensitive element may be a charge-coupled device (charge coupled device, CCD) or a complementary metal-oxide-semiconductor (complementary metal-oxide-semiconductor, CMOS) photoelectric transistor. The photosensitive element converts an optical signal into an electrical signal, and then transmits the electrical signal to the ISP for converting the electrical signal into a digital image signal. The ISP outputs the digital image signal to the DSP for processing. The DSP converts the digital image signal into an image signal in a standard format such as an RGB format or a YUV format. In some embodiments, the electronic device 100 may include one or N cameras 193, where N is a positive integer greater than 1.

For example, in the method for assisting fitness provided in this application, the camera may collect a user movement video. The photosensitive element converts a collected optical signal into an electrical signal, and then transmits the electrical signal to the ISP for converting the electrical signal into a digital image signal. The ISP outputs the digital image signal to the DSP for related image processing.

The digital signal processor is configured to process a digital signal, and may process another digital signal in addition to the digital image signal. For example, when the electronic device 100 selects a frequency, the digital signal processor is configured to perform Fourier transformation and the like on frequency energy.

The video codec is configured to: compress or decompress a digital video. The electronic device 100 may support one or more video codecs. In this way, the electronic device 100 may play or record videos in a plurality of coding formats, for example, moving picture experts group (moving picture experts group, MPEG)-1, MPEG-2, MPEG-3, and MPEG-4.

The NPU is a neural network (neural-network, NN) computing processor, quickly processes input information by referring to a structure of a biological neural network, for example, by referring to a mode of transmission between human brain neurons, and may further continuously perform self-learning. The NPU can implement applications such as intelligent cognition of the electronic device 100, such as image recognition, facial recognition, speech recognition, and text understanding.

The external memory interface 120 may be configured to connect to an external storage card, for example, a micro SD card, to extend a storage capability of the electronic device 100. The external storage card communicates with the processor 110 through the external memory interface 120, to implement a data storage function. For example, files such as music and videos are stored in the external storage card.

The internal memory 121 may be configured to store computer executable program code. The executable program code includes instructions. The processor 110 runs the instructions stored in the internal memory 121, to perform various function applications of the electronic device 100 and data processing. The internal memory 121 may include a program storage area and a data storage area. The program storage area may store an operating system, an application required by at least one function (for example, a sound playing function or an image playing function), and the like. The data storage area may store data (for example, audio data and a phone book) created during use of the electronic device 100, and the like. In addition, the internal memory 121 may include a high-speed random access memory, or may include a nonvolatile memory, for example, at least one magnetic disk storage device, a flash memory device, or a universal flash storage (universal flash storage, UFS).

The electronic device 100 may implement audio functions such as music playing and recording functions through the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The audio module 170 may be further configured to code and decode an audio signal. In some embodiments, the audio module 170 may be disposed in the processor 110, or some functional modules of the audio module 170 are disposed in the processor 110.

The speaker 170A, also referred to as a "horn", is configured to convert an electrical audio signal into a sound signal. The electronic device 100 may listen to music or answer a hands-free call by using the speaker 170A.

The receiver 170B, also referred to as an "earpiece", is configured to convert an audio electrical signal to a sound signal. When a call is answered or a voice message is listened to by using the electronic device 100, the receiver 170B may be put close to a human ear to listen to a voice.

The microphone 170C, also referred to as a "mike" or a "microphone", is configured to convert a sound signal into an electrical signal. When making a call or sending a voice message, a user may make a sound near the microphone 170C, to enter a sound signal to the microphone 170C. At least one microphone 170C may be disposed in the electronic device 100. In some other embodiments, two microphones 170C may be disposed in the electronic device 100, to collect a sound signal and implement a noise reduction function. In some other embodiments, three, four, or more microphones 170C may alternatively be disposed in the electronic device 100, to collect a sound signal, implement noise reduction, and identify a sound source, so as to implement a directional recording function and the like.

The headset jack 170D is configured to connect to a wired headset. The headset jack 170D may be the USB interface 130, or may be a 3.5 mm open mobile electronic device platform (open mobile terminal platform, OMTP) standard interface or a cellular telecommunications industry association of the USA (cellular telecommunications industry association of the USA, CTIA) standard interface.

The pressure sensor 180A is configured to sense a pressure signal, and may convert the pressure signal into an electrical signal. In some embodiments, the pressure sensor 180A may be disposed on the display 194. There are many types of pressure sensors 180A such as a resistive pressure sensor, an inductive pressure sensor, and a capacitive pressure sensor. The capacitive pressure sensor may include at least two parallel plates made of conductive materials. Capacitance between electrodes changes when force is applied to the pressure sensor 180A. The electronic device 100 determines pressure intensity based on a change of the capacitance. When a touch operation is performed on the display 194, the electronic device 100 detects a strength of the touch operation based on the pressure sensor 180A. The electronic device 100 may also calculate a touch location based on a detection signal of the pressure sensor 180A. In some embodiments, touch operations that are applied to a same touch position but have different touch operation strength may correspond to different operation instructions. For example, when a touch operation whose touch operation intensity is less than a first pressure threshold is performed on an icon of Messages, an instruction for viewing an SMS message is executed. When a touch operation whose touch operation intensity is greater than or equal to the first pressure threshold is performed on an icon of Messages, an instruction for creating an SMS message is executed.

The gyroscope sensor 180B may be configured to determine a motion posture of the electronic device 100. In some embodiments, an angular velocity of the electronic device 100 around three axes (namely, axes X, Y, and Z) may be determined by using the gyroscope sensor 180B. The gyroscope sensor 180B may be configured to implement image stabilization during photographing. For example, when a shutter is pressed, the gyroscope sensor 180B detects an angle at which the electronic device 100 jitters, and calculates, based on the angle, a distance for which a lens module needs to compensate, and allows the lens to eliminate the jitter of the electronic device 100 through a reverse motion, to implement image stabilization. The gyroscope sensor 180B may be further used in a navigation scenario and a somatic game scenario.

The barometric pressure sensor 180C is configured to measure barometric pressure. In some embodiments, the electronic device 100 calculates an altitude based on a barometric pressure value measured by the barometric pressure sensor 180C, to assist in positioning and navigation.

The magnetic sensor 180D includes a Hall sensor. The electronic device 100 may detect opening and closing of a flip cover by using the magnetic sensor 180D. In some embodiments, when the electronic device 100 is a clamshell phone, the electronic device 100 may detect opening and closing of a flip cover by using the magnetic sensor 180D, to set a feature such as automatic unlocking through flipping based on a detected opening or closing state of the flip cover.

The acceleration sensor 180E may detect magnitude of accelerations in various directions (usually on three axes) of the electronic device 100, and may detect a magnitude and a direction of gravity when the electronic device 100 is still. The acceleration sensor 180E may be further configured to identify a posture of the electronic device, and is applied to an application such as switching between a landscape mode and a portrait mode or a pedometer.

The distance sensor 180F is configured to measure a distance. The electronic device 100 may measure the distance in an infrared or a laser manner. In some embodiments, in a photographing scenario, the electronic device 100 may measure a distance by using the distance sensor 180F, to implement quick focusing.

The optical proximity sensor 180G may include, for example, a light-emitting diode (LED) and an optical detector such as a photodiode. The light-emitting diode may be an infrared light-emitting diode. The electronic device 100 emits infrared light by using the light-emitting diode. The electronic device 100 detects infrared reflected light from a nearby object by using the photodiode. When detecting sufficient reflected light, the electronic device 100 may determine that there is an object near the electronic device 100. When detecting insufficient reflected light, the electronic device 100 may determine that there is no object near the electronic device 100. The electronic device 100 may detect, by using the optical proximity sensor 180G, that the user holds the electronic device 100 close to an ear to make a call, to automatically perform screen-off for power saving. The optical proximity sensor 180G may also be used in a smart cover mode or a pocket mode to automatically perform screen unlocking or locking.

The ambient light sensor 180L is configured to sense ambient light luminance. The electronic device 100 may adaptively adjust brightness of the display 194 based on the sensed ambient light brightness. The ambient light sensor 180L may be further configured to automatically adjust a white balance during photographing. The ambient light sensor 180L may also cooperate with the optical proximity sensor 180G to detect whether the electronic device 100 is in a pocket, to avoid an accidental touch.

The fingerprint sensor 180H is configured to collect a fingerprint. The electronic device 100 may use a feature of the collected fingerprint to implement fingerprint-based unlocking, application lock access, fingerprint-based photographing, fingerprint-based call answering, and the like.

The temperature sensor 180J is configured to detect a temperature. In some embodiments, the electronic device 100 executes a temperature processing policy based on the temperature detected by the temperature sensor 180J. For example, when the temperature reported by the temperature sensor 180J exceeds a threshold, the electronic device 100 degrades performance of a processor near the temperature sensor 180J, to reduce power consumption for thermal protection. In some other embodiments, when the temperature is lower than another threshold, the electronic device 100 heats up the battery 142, to avoid abnormal shutdown of the electronic device 100 caused by a low temperature. In some other embodiments, when the temperature is lower than still another threshold, the electronic device 100 boosts an output voltage of the battery 142 to avoid abnormal shutdown caused by a low temperature.

The touch sensor 180K is also referred to as a "touch panel". The touch sensor 180K may be disposed on the display 194, and the touch sensor 180K and the display 194 form a touchscreen, which is also referred to as a "touch screen". The touch sensor 180K is configured to detect a touch operation on or near the touch sensor 180K. The touch sensor may transfer the detected touch operation to the application processor, to determine a type of a touch event. The display 194 may provide a visual output related to the touch operation. In some other embodiments, the touch sensor 180K may alternatively be disposed on a surface of the electronic device 100 in a position different from that of the display 194.

The bone conduction sensor 180M may obtain a vibration signal. In some embodiments, the bone conduction sensor 180M may obtain a vibration signal of a vibration bone of a human vocal part. The bone conduction sensor 180M may also be in contact with a human pulse, and receive a blood pressure beating signal. In some embodiments, the bone conduction sensor 180M may alternatively be disposed in a headset, to obtain a bone conduction headset. The audio module 170 may obtain a voice signal through parsing based on the vibration signal that is of the vibration bone of the vocal part and that is obtained by the bone conduction sensor 180M, to implement a voice function. The application processor may parse heart rate information based on the blood pressure beating signal obtained by the bone conduction sensor 180M, to implement a heart rate detection function.

The button 190 includes a power button, a volume button, and the like. The button 190 may be a mechanical button, or may be a touch button. The electronic device 100 may receive a button input, and generate a button signal input related to a user setting and function control of the electronic device 100.

The motor 191 may generate a vibration prompt. The motor 191 may be configured to provide an incoming call vibration prompt or a touch vibration feedback. For example, touch operations performed on different applications (for example, photographing and audio playing) may correspond to different vibration feedback effects. The motor 191 may also correspond to different vibration feedback effects for touch operations performed on different areas of the display 194. Different application scenarios (for example, a time reminder scenario, an information receiving scenario, an alarm clock scenario, and a game scenario) may also correspond to different vibration feedback effects. A touch vibration feedback effect may alternatively be customized. A touch vibration feedback effect may alternatively be customized.

The indicator 192 may be an indicator light, and may be configured to indicate a charging status and a power change, or may be configured to indicate a message, a missed call, a notification, and the like.

The SIM card interface 195 is configured to connect to a SIM card. The SIM card may be inserted into the SIM card interface 195 or detached from the SIM card interface 195, to implement contact with or separation from the electronic device 100. The electronic device 100 may support one or N SIM card interfaces, where N is a positive integer greater than 1. The SIM card interface 195 may support a nano-SIM card, a micro-SIM card, a SIM card, and the like. A plurality of cards may be simultaneously inserted into one SIM card interface 195. The plurality of cards may be of a same type, or may be of different types. The SIM card interface 195 may be further compatible with different types of SIM cards. The SIM card interface 195 may be further compatible with the external memory card. The electronic device 100 interacts with a network through the SIM card, to implement functions such as calling and data communication. In some embodiments, the electronic device 100 uses an eSIM, namely, an embedded SIM card. The eSIM card may be embedded in the electronic device 100, and cannot be separated from the electronic device 100.

A software system of the electronic device 100 may use a layered architecture, an event-driven architecture, a microkernel architecture, a micro service architecture, or a cloud architecture. In the embodiments of this application, an Android system with a layered architecture is used as an example to describe a software structure of the electronic device 100.

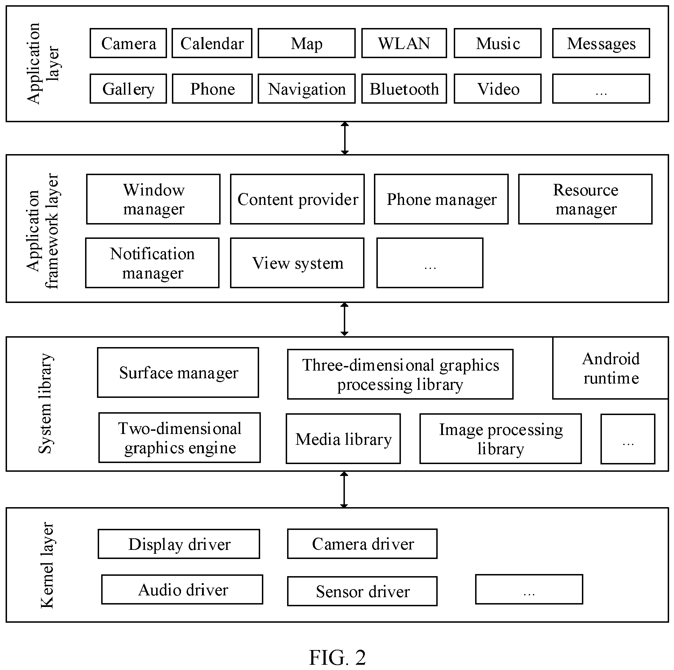

FIG. 2 is a block diagram of the software structure of the electronic device 100 according to this embodiment of this application. In the layered architecture, software is divided into several layers, and each layer has a clear role and task. The layers communicate with each other through a software interface. In some embodiments, an Android system is divided into four layers: an application layer, an application framework layer, an Android runtime (Android runtime) and system library, and a kernel layer from top to bottom. The application layer may include a series of application packages.

As shown in FIG. 2, the application packages may include applications such as Camera, Gallery, Calendar, Phone, Map, Navigation, WLAN, Bluetooth, Music, Video, and Messages.

The application framework layer provides an application programming interface (application programming interface, API) and a programming framework for an application at the application layer. The application framework layer includes some predefined functions.

For example, in this application, an image processing algorithm and the like may be included in the application framework layer.

As shown in FIG. 2, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to store and obtain data, and enable the data to be accessed by an application. The data may include a video, an image, audio, calls that are made and received, a browsing history and bookmarks, a phone book, and the like.

For example, in this application, the content controller may obtain, in real time, an image collected in a preview interface, and display a processed image in the preview interface.

The view system includes visual controls such as a control for displaying a text and a control for displaying an image. The view system may be configured to construct an application. A display interface may include one or more views. For example, a display interface including a notification icon of Messages may include a text display view and a picture display view.

For example, in this application, content such as "a user movement video", "a standard fitness movement", and "guidance information" displayed on the interface of the display may be displayed by the view system after the view system receives an instruction of the processor.

The phone manager is configured to provide a communications function of the electronic device 100, for example, management of a call status (including answering or declining a call).

The resource manager provides various resources for an application, such as a localized character string, an icon, a picture, a layout file, and a video file.

The notification manager enables an application to display notification information in a status bar, and may be configured to convey a notification message. The notification manager may automatically disappear after a short pause without requiring a user interaction. For example, the notification manager is configured to provide notifications of download completing, a message prompt, and the like. The notification manager may alternatively be a notification that appears in a top status bar of the system in a form of a graph or a scroll bar text, for example, a notification of an application running on the background or a notification that appears on the screen in a form of a dialog window. For example, text information is displayed in the status bar, an alert sound is played, the electronic device vibrates, or the indicator light blinks.

The Android runtime includes a kernel library and a virtual machine. The Android runtime is responsible for scheduling and managing the Android system.

The kernel library includes two parts: a function that needs to be invoked in Java language and a kernel library of Android.

The application layer and the application framework layer run on the virtual machine. The virtual machine executes Java files at the application layer and the application framework layer as binary files. The virtual machine is configured to implement functions such as object lifecycle management, stack management, thread management, security and exception management, and garbage collection.

The system library may include a plurality of function modules, for example, a surface manager (surface manager), a media library (media library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playing and recording of a plurality of commonly used audio and video formats, static image files, and the like. The media library may support a plurality of audio and video coding formats such as MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

For ease of understanding, in the following embodiments of this application, an electronic device having the structures shown in FIG. 1 and FIG. 2 is used as an example to describe in detail, with reference to the accompanying drawings and application scenarios, the method for assisting fitness provided in the embodiments of this application.

The method for assisting fitness provided in the embodiments of this application may be applied to an electronic device such as a television, a mobile phone, a tablet computer, a wearable device, a vehicle-mounted device, an augmented reality (augmented reality, AR)/virtual reality (virtual reality, VR) device, a notebook computer, an ultra-mobile personal computer (ultra-mobile personal computer, UMPC), a netbook, or a personal digital assistant (personal digital assistant, PDA). A specific type of the electronic device is not limited in the embodiments of this application.

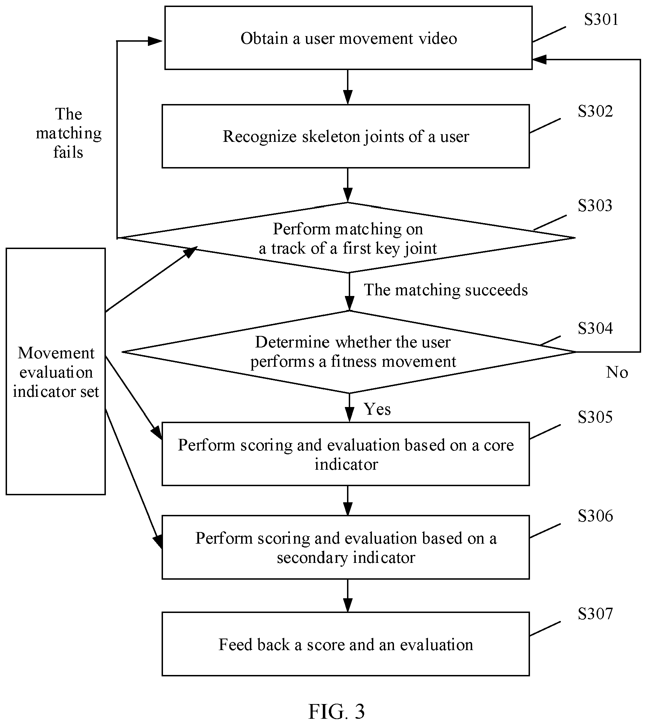

FIG. 3 is a schematic flowchart of a method for assisting fitness according to an embodiment of this application.

Step S301: An electronic device obtains a user movement.

The user movement may be a user movement in a real-time video collected by a camera. In other words, the electronic device may obtain the user movement collected by the camera. The video that includes the user movement and that is obtained by the electronic device may be referred to as a user movement video.

Before step S302 or step S303, a user may select a specific fitness movement from at least one fitness movement in a movement evaluation indicator set. In subsequent steps, processing is performed on the user movement based on the specific fitness movement. The movement evaluation indicator set may be determined based on professional knowledge. That is, the electronic device may obtain input information. The input information is used to indicate the fitness movement.

Step S302: The electronic device recognizes skeleton joints of the user in the user movement.

The electronic device may recognize skeleton joints of the user in each frame of image in the user movement, or may recognize skeleton joints of the user at fixed or unfixed intervals of a plurality of frames of images in the user movement. A skeleton joint of the user may be used to represent a body part of the user.

The skeleton joints of the user may include all skeleton joints on the user body in an image. Alternatively, the skeleton joints of the user may include one or more of a first key joint corresponding to a first standard joint of the specific fitness movement in the movement evaluation indicator set, a second key joint corresponding to a second standard joint, a third key joint corresponding to a third standard joint, and a fourth key joint corresponding to a fourth standard joint. The first key joint, the second key joint, the third key joint, and the fourth key joint are all skeleton joints on the user body in the image.

Step S303: The electronic device performs matching on a track of the first key joint.

The electronic device performs matching on the track of the first key joint in the skeleton joints of the user based on the movement evaluation indicator set.

Before step S303, the electronic device may further determine at least one of a horizontal direction and a vertical direction of the user movement. Directions of a location of a body part of the user, a change of the location, a motion track of the body part, and the like may be determined based on the horizontal direction or the vertical direction. An apparatus such as a gyroscope sensor in the electronic device may be used to determine the horizontal direction and the vertical direction of the user movement in a process of collecting the user movement. For an electronic device such as a television or a desktop computer that does not frequently move, a default horizontal direction or a default vertical direction may be set for a collected image. The horizontal direction and the vertical direction of the user movement may be determined based on at least one of the default horizontal direction and the default vertical direction.

For the specific fitness movement, the expert may determine the at least one first standard joint and a motion track of each of the at least one first standard joint in the fitness movement based on professional knowledge, and store the motion track of the first standard joint in the movement evaluation indicator set. The movement evaluation indicator set includes a correspondence between at least one fitness movement and a motion track of the at least one first standard joint. The motion track of the first standard joint is a motion track of a skeleton joint on a human body in a standard movement of the specific fitness movement. The skeleton joint of the human body includes the first standard joint. The first standard joint may be understood as a joint of a body part of the user.

The first key joint is a skeleton joint corresponding to the first standard joint in the user movement, or the first key joint is a skeleton joint at a same location as the first standard joint in the user movement.

In some embodiments, the electronic device may compare the motion track of the first key joint with the motion track of the first standard joint. If a similarity between the motion track of the first standard joint and the motion track of the first key joint is greater than a preset similarity, the matching fails, and it is considered that the user does not perform the fitness movement, and step S301 is performed to reobtain a user movement. If the similarity between the motion track of the first standard joint and the motion track of the first key joint is less than or equal to the preset similarity, the matching succeeds.

In some other embodiments, the electronic device may determine whether the motion track of the first key joint meets a preset feature, that is, whether the motion track of the first key joint has the preset feature. The preset feature may also be understood as a preset condition. The electronic device may determine, from the user movement, a candidate movement that meets a preset feature of a motion track of a body part corresponding to the first standard joint.

The preset feature may be a shape of the motion track, a cyclicality of the motion track, or the like. For example, the preset feature may be a cyclic location change rule. A preset cyclic location change rule of the first standard joint may also be understood as a cyclic location change rule of the first standard joint.

The electronic device may determine the candidate movement based on a location change cyclicality of the first standard joint. Different fitness movements may correspond to a same candidate movement selection rule or different candidate movement selection rules. According to a candidate movement selection rule, the electronic device may use, as the candidate movement, images of a video that is in a cycle of the first key joint and that corresponds to the motion track of the first key joint, that is, determine the candidate movement of the user in the candidate movement. Alternatively, the electronic device may use, as the candidate movement, images of a video that is in the user movement and that corresponds to a point or a range in the motion track of the first key joint.

The movement evaluation indicator set may include a preset feature, and the preset feature is used to indicate a location change mode of the first standard joint. The location change mode of the first standard joint may be a motion direction of the first standard joint, for example, an upward motion or a downward motion, or a full process or an upward motion in an up-and-down reciprocation motion process. The location change mode of the first standard joint may alternatively be a shape of the motion track. For example, the motion track is in a shape such as a triangle, a circle, an arc, or a broken line. The location change mode of the first standard joint may alternatively be a part of a complete shape completed by the first standard joint. For example, the track of the first standard joint is a triangle, and the location change mode is a motion performed by the first standard joint on an edge.

The determined candidate movement may be, for example, a user movement corresponding to an upward motion of the first key joint in an up-and-down reciprocation motion process. Alternatively, when the motion track of the first key joint is a triangle, the determined candidate movement is a corresponding user movement when the first key joint moves on an edge of the triangle.

After the candidate movement is determined, step S304 is performed. In steps S304 to S307, the electronic device performs processing on the candidate movement.

For example, in a squat movement, the first standard joint may be a hip joint. The electronic device may recognize a hip joint of the user in each frame of image in the user movement as the first key joint. During the squat movement, a height of the hip joint fluctuates cyclically. The electronic device may match the motion track of the first key joint with the motion track of the first standard joint, determine a start point and an end point of a cycle of an up-and-down motion of the hip joint, and use a video between the start point and the end point of the cycle as the candidate movement. Alternatively, the electronic device uses a video of an upward motion or a downward motion of the hip joint in a cycle as the candidate movement. That is, the preset feature of the motion track may be a motion in a cycle of the up-and-down motion of the hip joint, or may be an upward motion of the hip joint in a cycle of the up-and-down motion of the hip joint.

For a specific fitness movement, a trigger condition is that the motion track of the first key joint successfully matches the motion track of the first standard joint. When the trigger condition is met, step S304 is performed.

Step S304: The electronic device determines that the user performs the fitness movement.

The electronic device may determine, based on a recognition condition, whether the user movement in the user movement is the specific fitness movement. The recognition condition is a preset condition.

The determined candidate movement is processed, to determine whether the candidate movement of the user in the candidate movements meets a recognition condition of the specific movement in the movement evaluation indicator set. When the candidate movement meets the recognition condition, it is determined that the candidate movement is the specific movement performed by the user. When the candidate movement does not meet the recognition condition, it is determined that the candidate movement is not the specific movement performed by the user.

The recognition condition includes a condition meet by the second key joint corresponding to the second standard joint. The at least one second standard joint may include all or some of the at least one first standard joint, and the at least one second standard joint may also include another skeleton joint. The recognition condition may include location change information of the second standard joint. The location change information of the second standard joint may include a location change range of the second standard joint in the video. The location change information of the second standard joint may alternatively be a change range of relative locations of the second standard joint.

The location change information of the second standard joint may be used to indicate a movement change range of a body part. The movement change range of the body part may include an angle change range of the body part, or may include a change range of relative locations of body parts. The angle change range of the body part may be understood as an interval range between a largest value and a smallest value of an included angle between the body part and the horizontal direction or the vertical direction in the user movement. The change range of relative locations of body parts may be understood as an interval range between a largest value and a smallest value of a distance between the body parts. A distance between two body parts may be determined based on lengths of the body parts. For example, the distance between the two body parts is a multiple or a proportion of a length of one of the body parts.

The recognition condition may be determined based on professional knowledge. The second key joint is a skeleton joint corresponding to the second standard joint in the user movement, or the second key joint is a skeleton joint at a same location as the second standard joint in the user movement.

For example, for the squat movement, the thigh gradually changes from being parallel to the vertical direction to being parallel to the horizontal direction as the height of the hip joint moves from crest to trough. A recognition condition of the squat movement may include: A change of a thigh angle meets a first preset range. The second standard joint may include the hip joint and joints (namely, a knee joint and a hip joint) of two ends of the thigh, and the thigh angle may be determined based on the joints of the two ends of the thigh in the candidate movement. In other words, when the change of the thigh angle in the candidate movement meets the first preset range, it is considered that the user performs the squat movement.

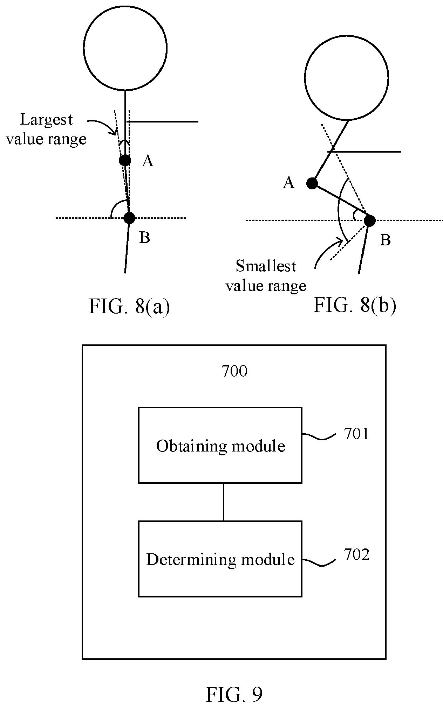

Refer to FIG. 8(a) and FIG. 8(b). It is determined that the user performs the squat movement. In FIG. 8(a), the user stands, and in FIG. 8(b), the user squats to the lowest. During the squat movement, a hip joint A of the user moves up and down, and an included angle between the thigh and the horizontal direction changes. That is, the thigh angle changes. A movement change of the thigh is reflected by the change of the thigh angle.

A preset feature of the squat movement in the movement evaluation indicator set may be a downward motion in an up-and-down motion of the hip joint A. The candidate movement in the user movement may be determined based on the preset feature.

The recognition condition of the squat movement in the movement evaluation indicator set may include a range of an amplitude of the thigh angle.

The electronic device may determine whether the amplitude of the thigh angle in the candidate movement meets the recognition condition, to determine whether to output guidance information.

The movement evaluation indicator set may include a location that is in a motion track and that corresponds to a largest value of the thigh angle, and a location that is in a motion track and that corresponds to a smallest value of the thigh angle. Alternatively, the movement evaluation indicator set may include a location that is of the hip joint, that is in the motion track, and that corresponds to a movement start location of a thigh movement, or the movement evaluation indicator set may include a location that is of the hip joint, that is in the motion track, and that corresponds to a movement end location of a thigh movement. It may be determined, based on the motion track of the hip joint A, that a thigh location in the first frame of image of the candidate movement is the movement start location of the thigh, and a thigh location in the last frame of image of the candidate movement is the movement end location of the thigh.

The electronic device may determine the thigh angle based on the hip joint A and a knee joint B. During the user movement, the electronic device determines whether the movement change of the thigh meets a largest value requirement and a smallest value requirement of a preset movement change range. In other words, the electronic device determines a movement change range of the thigh, where a largest value of the range meets the largest value requirement of the preset movement change range, and a smallest value of the range meets the smallest value requirement of the preset movement change range.

The largest value requirement may be an interval. That is, the largest value of the movement change range of the thigh that is determined by the electronic device falls within a largest value interval of the preset movement change range. The smallest value requirement may also be an interval. That is, the smallest value of the movement change range of the thigh that is determined by the electronic device falls within a smallest value interval of the preset movement change range.

In the figure, dashed lines indicate a movement direction range of the thigh corresponding to the smallest value range of the preset movement change range, and a movement direction range of the thigh corresponding to the largest value range of the preset motion change range. In the user movement, when an angle between the thigh and the ground is the smallest, a direction of the thigh falls within the movement direction range of the thigh corresponding to the smallest value range, and when the angle between the thigh and the ground is the largest, a direction of the thigh falls within the movement direction range of the thigh corresponding to the largest value range. In this case, the electronic device may determine that the user performs the squat movement.

For the user in the figure, the largest value of the movement change of the thigh falls within the largest value range of the preset motion change range, and the smallest value of the movement change of the thigh falls within the smallest value range of the preset movement change range. That is, the movement change of the thigh of the user meets the largest value requirement and the smallest value requirement of the preset movement change range. If there is no other recognition condition, it may be determined that the user in the figure is performing the squat movement.

The recognition condition of the squat movement may further include a change range of a relative distance of the hip joint to the ankle joint. The second standard joints may include the hip joint, the knee joint, and the ankle joint. The change range of the relative distance of the hip joint to the ankle joint is determined based on a distance between the hip joint and the knee joint and a distance between the knee joint and the ankle joint. The relative distance of the hip joint to the ankle joint may be obtained by dividing a distance between the hip joint and the ankle joint by a sum of the distance between the hip joint and the knee joint and the distance between the knee joint and the ankle joint. That is, the relative distance of the hip joint to the ankle joint may be represented by using a ratio of the distance between the hip joint and the ankle joint to a leg length. An interval range between a smallest value and a largest value of the ratio in the user movement is the change range of the relative distance of the hip joint to the ankle joint.

If a determining result is that the user performs the fitness movement, step S305 is performed. Otherwise, step S301 is performed to reobtain a user movement.

When it is determined that the user performs the fitness movement, different guidance systems may be designed for different fitness movements, to evaluate a standard level of the fitness movement. A guidance system for each movement may include a core indicator and a secondary indicator. The core indicator is used to determine a basic score and guidance for a user movement, and the secondary indicator is used to revise the score and provide comprehensive guidance.

Step S305: The electronic device performs scoring and evaluation based on the core indicator.

The movement evaluation indicator set includes the core indicator. The core indicator may include an evaluation standard for location information of some body parts. The body parts are represented by skeleton joints. That is, the core indicator may include an evaluation standard for a third standard joint. A score and an evaluation corresponding to the third standard joint in the user movement are obtained based on the core indicator.

Location information of a body part may include one or more of a movement change range of the body part, a limit value of the movement change range of the body part, and a motion track of the body part. The limit value of the movement change range of the body part is a largest value or a smallest value of the movement change range of the body part.

The core indicator may include a correspondence between a movement change range of a body part and evaluation information, or may include a correspondence between a limit value of a movement change range of a body part and evaluation information, or may include a correspondence between a motion track of a body part and evaluation information.

To perform scoring and evaluation based on the core indicator, the electronic device may score and evaluate relative locations, a location change status, and the like of the third key joint that is in the candidate video and that corresponds to the third standard joint. The third key joint is a skeleton joint corresponding to the third standard joint in the user movement, or the third key joint is a skeleton joint at a same location as the third standard joint in the user movement.

The core indicator may include a correspondence between a plurality of types of location information of a third standard joint and a plurality of scores and/or evaluations. The core indicator may also include a correspondence between a plurality of types of location information of a third standard joint and a plurality of score increments or decrements, and/or the core indicator may include a correspondence between a plurality of types of location information of a third standard joint and a plurality of evaluations. The score may be determined based on the location information of the third standard joint by increasing or decreasing a preset score. The evaluation can also be understood as guidance and suggestions.

The location information of the third standard joint may include relative locations of the third standard joints and/or a location change status of each third standard joint. The at least one third standard joint may include all or some of the at least one first standard joint, the at least one third standard joint may include all or some of the at least one second standard joint, the at least one third standard joint may also include another skeleton joint. The third standard joints may be some of the at least one first standard joint and the at least one second standard joint.

The electronic device may determine one or more frames of images in the candidate movement or the user movement based on the motion track of the first key joint. The electronic device may determine one or more frames of images in the candidate movement or the user movement based on a motion track of the third key joint. The electronic device may obtain a corresponding score or evaluation based on relative locations of the third key joint in the one or more frames of images. The electronic device may determine a selection manner of the one or more frames of images based on the fitness movement. The relative locations of the third key joint may reflect a location of a body part of the user, for example, an angle of the body part or a distance between body parts.

The electronic device may obtain, based on the motion track of the third key joint, a score and/or an evaluation corresponding to the motion track of the third key joint.

The electronic device may determine a location change range of the third key joint in the candidate movement or the user movement. A score and/or an evaluation corresponding to the location change range of the third key joint or a limit value of the change range may be obtained.

For example, for the squat movement, the core indicator may include a correspondence between a smallest value of a thigh angle range and a score, and a correspondence between a thigh angle range and an evaluation. When a smallest value of an angle between the thigh and the ground is less than 75 degrees, it is considered that the user completes a squat movement, and when the angle is 0 degrees, it is considered that a best completion rate is achieved for the indicator. When the smallest value of the angle between the thigh and the ground (namely, the horizontal direction) is less than 75 degrees, there may be several intervals (for example, four intervals: less than 0 degrees, 0 degrees to 25 degrees (excluding 25 degrees), 25 degrees to 50 degrees (excluding 50 degrees), and 50 degrees to 75 degrees (excluding 75 degrees)). Each interval corresponds to a different score, and each interval corresponds to a same evaluation or a different evaluation. When the angle between the thigh and the ground determined based on the third key joint is a value, a range to which the value belongs is determined, and a corresponding score and evaluation are determined, to provide corresponding guidance for the user (for example, when the smallest value of the angle between the thigh and the ground determined based on the third key joint is 32 degrees and belongs to the interval 25 degrees to 50 degrees, a score corresponding to the interval is 80, and an evaluation corresponding to the interval is: squat lower).

Step S306: The electronic device performs scoring and evaluation based on the secondary indicator.

The movement evaluation indicator set includes the secondary indicator. The secondary indicator may include an evaluation standard for a fourth standard joint. To perform scoring and evaluation based on the secondary indicator, the electronic device may score and evaluate relative locations, a location change status, and the like of the fourth key joint in the candidate video. The fourth key joint is a skeleton joint corresponding to the fourth standard joint in the user movement, or the fourth key joint is a skeleton joint at a same location as the fourth standard joint in the user movement.

The secondary indicator may include a correspondence between location information of the fourth standard joint and a score decrement and/or an evaluation. It may also be understood that the secondary indicator includes a correspondence between location information of a body part corresponding to the fourth standard joint and a score decrement and/or an evaluation. When the user movement does not meet a threshold of the secondary indicator, the score calculated based on the core indicator may be decreased and corresponding guidance may be determined.

The location information of the fourth standard joint may include relative locations of the fourth standard joint and/or a location change status of each fourth standard joint. The at least one fourth standard joint may include all or some of the at least one third standard joint, and the at least one fourth key joint may also include a skeleton joint other than the third standard joint. The location change may be, for example, a location change amplitude, a location change range, or a motion track.

For example, for the squat movement, the secondary indicator may include a correspondence between a shin angle range and a score, and a correspondence between a shin angle range and an evaluation. The fourth standard joints may include the knee joint and the ankle joint. The electronic device may determine a shin angle based on relative locations of the knee joint and the ankle joint. The secondary indicator may alternatively include a correspondence between a torso angle range and a score, and a correspondence between a torso angle range and an evaluation. The secondary indicator may include: An angle between the shin and the ground is greater than 50 degrees. When the candidate movement does not meet the secondary indicator, the user can be provided with a corresponding evaluation for guidance, for example, prompting the user not to lean the shin forward too much.

For different fitness movements, evaluation indicators may be completely different, and impact of a movement of each body part in different fitness movements is also different (for example, a movement of an arm in the squat movement is usually unrelated, while in a dumbbell curl movement, a movement of an arm is a core, and movements of legs are not important). If determining, evaluation, and guidance are performed on a movement of each body part, excessive useless information is transmitted to the user, and user experience is affected. After steps S305 and S306, an evaluation corresponding to a specific fitness exercise can be determined, and effective guidance can be provided for the user.

Step S307: The electronic device feeds back the score and the evaluation.

The electronic device may output feedback information, to feed back the score and the evaluation to the user, where the feedback information includes the score determined in step S306 and the evaluation determined in steps S305 and S306. For example, the score and the evaluation may be fed back in a manner such as popping up a text on a screen, or playing a prompt voice.

When a use scenario of the user is not suitable for public voice playing, the electronic device may play the score and/or the evaluation via voice by using a Bluetooth headset, or display the score and/or evaluation on the screen. The public voice playing means playing by using the speaker.

For example, through image recognition, a plurality of persons are determined in the user movement, and the electronic device may display the score and/or the evaluation on the screen.

When it is determined that the score and/or the evaluation are/is not played by using the speaker, the electronic device may perform image recognition to determine whether the user performing the fitness movement wears the Bluetooth headset. When it is determined that the user wears the Bluetooth headset, and the Bluetooth headset is connected to the apparatus for assisting fitness over Bluetooth, the score and/or the evaluation are/is played by using the Bluetooth headset.

Alternatively, the electronic device may obtain feedback manner indication information, where the feedback manner indication information is used to indicate a feedback manner, namely, a manner of outputting the score and/or the evaluation.

The electronic device may count fitness movements of the user based on the motion track of the first key joint, and record a quantity of fitness movements completed by the user. The electronic device may store a correspondence between a quantity of cycles of the first standard joint and a quantity of fitness movements in the movement evaluation indicator set.

For example, one cycle of the motion track of the first key joint corresponds to one fitness movement completed by the user. For different fitness movements, a plurality of cycles of the motion track of the first key joint may correspond to one fitness movement completed by the user, or one cycle of the motion track of the first key joint may correspond to one fitness movement completed by the user.

Alternatively, the electronic device may count fitness movements of the user based on the motion track of the second key joint or the third key joint.

The electronic device may determine a plurality of candidate movements according to steps S301 to S304. The electronic device may determine, based on a quantity of candidate movements, a quantity of times that the user completes the fitness movement. The quantity of times of completing the fitness movement may be fed back to the user.

It is determined, based on an evaluation rule of a most core movement, namely, the recognition condition, that the user performs the specific fitness movement. When a user movement meets the recognition condition, even if a movement of another body part is not standard, it is still considered that the user is trying to learn the fitness movement, but the movement is not standard. However, if a user movement does not meet the recognition condition, even if a movement of a body part is very similar to a standard movement of the fitness movement, the user movement is not the fitness movement, to avoid false recognition of the fitness movement. Therefore, in this embodiment of this application, whether the user movement is the fitness movement can be accurately recognized. Even if a user movement is not standard, it can be determined, by using the method provided in this embodiment of this application, that the user performs the fitness movement.

By matching the motion track of the first key joint with the motion track of the first standard joint, the electronic device may determine start time and end time of the fitness movement performed by the user, to determine a user movement performed between the start time and the end time. The electronic device determines whether the user performs the fitness movement and scores the user movement. That is, the electronic device determines, based on a smallest completion indicator of the specific fitness movement, namely, the recognition condition, whether the user performs the fitness movement. A movement that does not meet the recognition condition is filtered out. That is, a user movement that is not the fitness movement is not scored and evaluated, but only a movement that meets the recognition condition is scored and evaluated, and guidance is provided for the user regarding the movement.

Due to an environmental limitation, some body parts may exceed the screen, that is, some skeleton joints of the user may not be included in the user movement.

Before step S303, the electronic device may determine whether the first key joints include all the first standard joints, and whether the second key joints include all the second standard joints.

If the first key joints include all of the first standard joints, and the second key joints include all of the second standard joints, step S303 is performed.

If the first key joints include no joint in the first standard joints, or the second key joints include no joint in the second standard joints, step S301 is performed to reobtain a user movement. In this case, the electronic device may output first prompt information, and the first prompt information is used to prompt the user to adjust a range that of the user body and that is collected in the user movement.

The first prompt information may be used to prompt the user to adjust a location of the user relative to the camera. The first prompt information may further include information about the first standard joint that is not included in the first key joints and/or the second standard joint that is not included in the second key joints, or the first prompt information may further include information about all the first standard joints and all the second standard joints, to prompt the user to adjust the location of the user relative to the camera. In this way, the user movement includes all of the first standard joints and the second standard joints, and the first standard joints and the second standard joints do not exceed a collection range of the user movement.

Before step S305, the electronic device may determine whether a third standard joint exists in the candidate movement, that is, whether the third key joints include all the third standard joints.

In some embodiments, if no joint in the third standard joints exists in the candidate movement, step S301 may be performed to reobtain a user movement. If all the third standard joints exist in the candidate movement, step S305 is performed. If the at least one third standard joint is all or some of the at least one first standard joint and the at least one second standard joint, when it is determined that the first key joints include all the first standard joints and the second key joints include all the second standard joints, the electronic device may determine that all the third standard joints exist in the candidate movement.

In some other embodiments, if no joint in the third standard joints exists, the electronic device may determine that the score of the candidate movement is a lowest score. If all or some of the at least one third standard joint exist, step S305 may be performed.

For the third standard joint that does not exist in the candidate movement, impact of the joint may be considered when a score is to be determined. That is, a score corresponding to the joint is deducted. An evaluation corresponding to the third standard joint that does not exist may no longer be determined.

If no third standard joint exists or some of the at least one third standard joint do not exist, second prompt information may be output, and the second prompt information is used to prompt the user to adjust the range that is of the user body and that is collected in the user movement. In other words, the second prompt information is used to prompt the user to adjust the location of the user relative to the camera. The second prompt information may include information about a third standard joint that is not included in the third key joints, to prompt the user to adjust the location of the user relative to the camera, so that the user movement includes all of the third standard joints, and a body part corresponding to the third standard joint does not exceed a spatial collection range of the user movement. The third key joints one-to-one correspond to the third standard joints.

Before step S306, the electronic device may determine whether a fourth standard joint exists in the candidate movement. If no joint in the fourth key joint exists, it may be determined that the score of the candidate movement is the lowest score. If all or some of the at least one fourth key joint exist, step S306 may be performed. When some fourth key joints do not exist, a total score may be appropriately decreased. The electronic device may decrease, based on score decrements that are in the movement evaluation indicator set and that correspond to the some fourth key joints that do not exist, a score calculated based on the core indicator.

If no fourth standard joint exists or some of the at least one fourth standard joint do not exist, the electronic device may output third prompt information. The third prompt information is used to prompt the user to adjust a collection range of the user body in the user movement. In other words, the third prompt information is used to prompt the user to adjust the location of the user relative to the camera. The third prompt information may include information about a fourth standard joint that is not included in the fourth key joints, to prompt the user to adjust the location of the user relative to the camera, so that the user movement includes all of the fourth standard joints, and the fourth standard joints do not exceed a collection range of the user movement.

For the fourth standard joint that does not exist in the candidate movement, namely, the fourth standard joint that is not included in the fourth key joints, the electronic device may consider impact of the joint when determining a score. That is, a score corresponding to the joint is deducted. The electronic device may no longer determine an evaluation corresponding to the fourth standard joint that does not exist.

The electronic device may output fourth prompt information. The fourth prompt information is used to prompt the user that the feedback information is incomplete and that all standard joints are not evaluated. A standard joint that is not evaluated may be a third standard joint or a fourth standard joint. In other words, when it can be recognized that the user makes the fitness movement, the electronic device may prompt the user when determining that some fourth standard joints are missed in information about the fourth key joints.

In the foregoing manner, a fault tolerance mechanism is provided when some body parts of the user are not in the user movement. For an image-based fitness assistance scenario, especially a use scenario at home, space is limited, and a location and an angle of an image obtaining device are usually fixed. As a result, in a process in which the user performs the fitness movement, some body parts may be outside the screen and cannot be recognized (for example, the ankle is outside the screen and cannot be seen because a close distance between the user and the device). When some body parts exceed a range, namely, a screen range or a video collection range of the user movement due to an environmental limit, the fitness movements performed by the user may still be counted, scored, and evaluated by using the method provided in this embodiment of this application, to avoid recognition and evaluation errors caused because some skeleton joints are not in the image recognition range.

For each fitness movement, a minimum joint set that can recognize the movement, namely, a joint set including the first key joints and the second key joints is created. When all joints in the minimum joint set exist in the user movement, the fitness movement performed by the user may be recognized, and fitness movements completed by the user may be counted.

For each fitness movement, a basic guidance joint set is created, that is, a joint set including the third standard joints. When all joints in the basic guidance joint set exist in the user movement, basic scoring and evaluation may be performed on the fitness movement performed by the user. The basic guidance joint set may be the same as or different from the minimum joint set. For the squat movement, the basic guidance joint set and the minimum joint set each may include the hip joint and the knee joint.

If the user movement includes all joints in the basic guidance joint set, the electronic device may provide basic scoring and guidance for the user.

If the user movement does not include all joints in the basic guidance joint set and/or the minimum joint set, the electronic device may output prompt information, to prompt the user to adjust a collection range of the user movement, so that the collected user movement includes all joints in the basic guidance joint set and/or the minimum joint set.

For each fitness movement, an extended guidance joint set is created, that is, a joint set including the fourth standard joints. When not all the fourth standard joints exist in the user movement, it cannot be determined whether the user movement meets the secondary indicator, an evaluation corresponding to the secondary indicator may not be output, and the score may be appropriately decreased.

After steps S301 to S307, it can be accurately determined whether the user makes the fitness movement, a quantity of times that the user completes the movement is recorded, quality of the movement completed by the user is evaluated, an incorrect partial movement is recognized, and feedback and guidance are provided for the user.

It should be understood that information such as a location change range and a motion track related to a location of a user body part may be used to indicate a location in the two-dimensional space, that is, a location in a user movement, or may be used to indicate a location in the three-dimensional space, that is, a location in the three-dimensional space determined based on the user movement. This is not limited in this embodiment of this application.

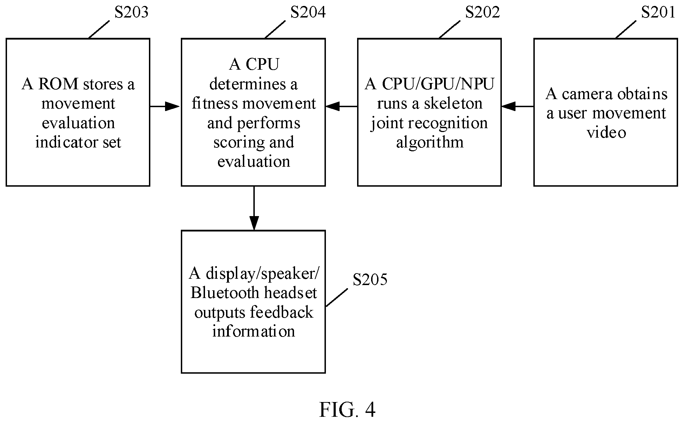

FIG. 4 shows a method for assisting fitness according to an embodiment of this application. The method may be performed by an apparatus for assisting fitness, and the apparatus for assisting fitness is an electronic device. The apparatus for assisting fitness includes a camera, a processor, a memory, a display/speaker/Bluetooth communications module, and the like. The processor includes a CPU, and may further include a GPU, an NPU, or the like.

Step S201: The camera obtains a user movement video.

Step S202: The CPU/GPU/NPU runs a skeleton joint recognition algorithm to recognize skeleton joints of a user in the user movement video.

Step S203: The memory stores a movement evaluation indicator set. The memory may be, for example, a ROM.

Step S204: The CPU determines a fitness movement and performs scoring and evaluation. The CPU determines, based on the movement evaluation indicator set stored in the memory and the recognized skeleton joints, whether the user performs the fitness movement, and scores and evaluates a user movement when determining that the user performs the fitness movement.

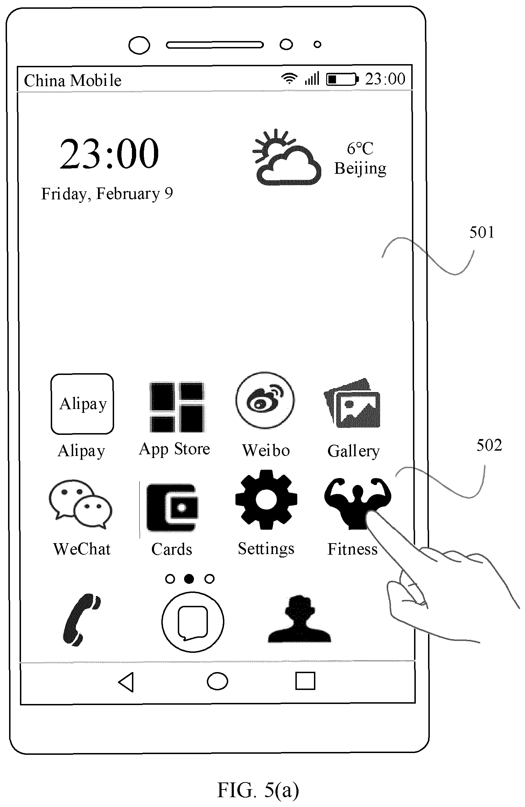

Step S205: The display/speaker/a Bluetooth headset outputs feedback information. The Bluetooth communications module may send the feedback information to the Bluetooth headset, and the Bluetooth headset may output the feedback information. The feedback information may include a score and an evaluation of the user movement. The display outputs the feedback information. For a graphical user interface, refer to FIG. 5(a) to FIG. 5(c).

FIG. 5(a) to FIG. 5(c) are schematic diagrams of graphical user interfaces (graphical user interfaces, GUIs) according to an embodiment of this application. In this application, an example in which the apparatus for assisting fitness is a mobile phone is used for description.

FIG. 5(a) shows currently output interface content 501 that is displayed by a screen display system of the mobile phone in an unlocking mode of the mobile phone. The interface content 501 is a home screen of the mobile phone. The interface content 501 displays a plurality of third-party applications (applications, apps), such as Alipay, App Store, Gallery, Weibo, WeChat, Cards, Settings, and Fitness. It should be understood that the interface content 501 may further include more applications. This is not limited in this application.

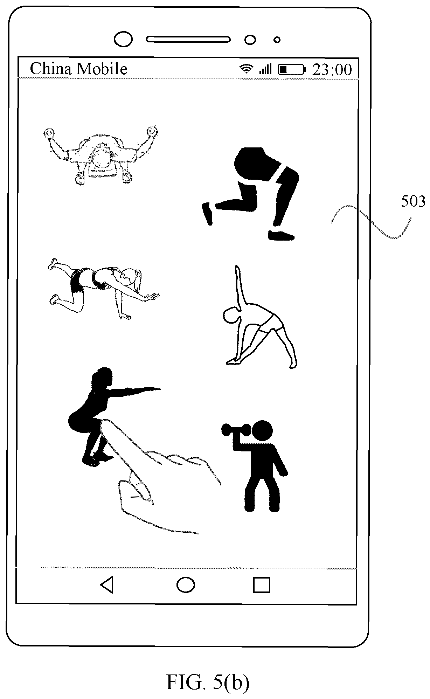

After detecting that a user taps an icon 502 of the fitness application on the home screen 501, the mobile phone may start the fitness application, and display an interface 503 of the fitness application shown in FIG. 5(b). The interface 503 of the fitness application may include a plurality of fitness movements.

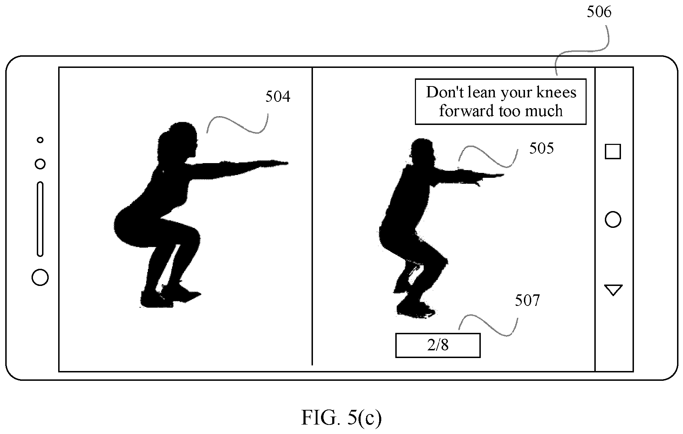

After detecting that the user taps a fitness movement in the interface 503 of the fitness application, the mobile phone may display a guidance interface of the fitness movement shown in FIG. 5 (c). The guidance interface may include a standard fitness movement 504, a user movement video 505 collected by the camera in real time, an evaluation, namely, guidance information 506, a user movement count 507, and the like.

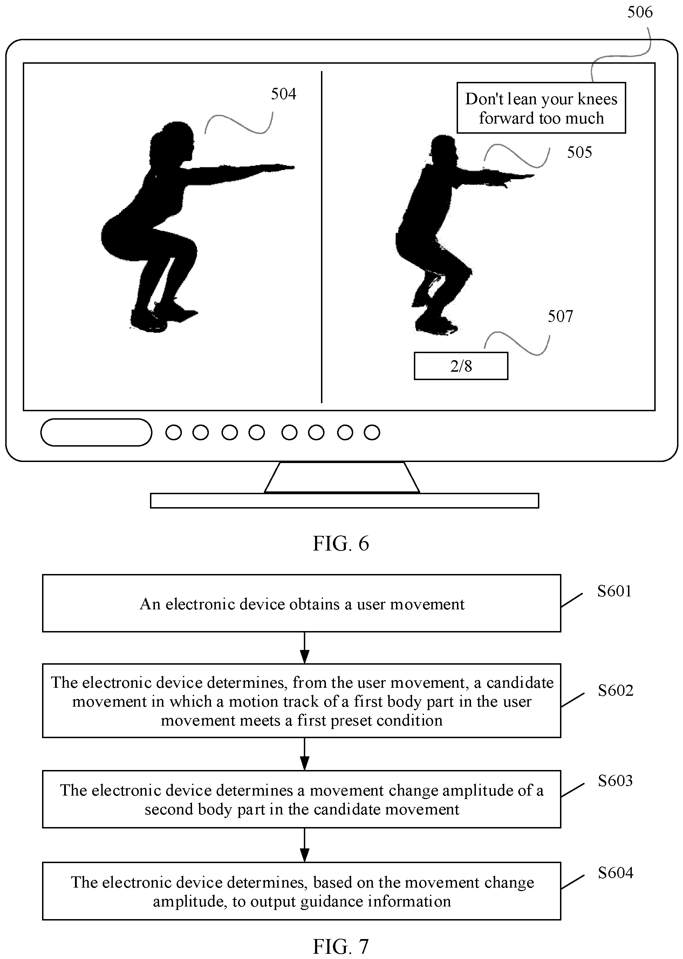

FIG. 6 is a schematic diagram of a graphical user interface according to an embodiment of this application. A television may display the guidance interface of the fitness movement shown in FIG. 6.

It should be understood that FIG. 5(a) to FIG. 5(c) and FIG. 6 are merely examples for description. Another electronic device with a display function, such as a tablet computer or a display of a personal computer, may also display the guidance interface of the fitness movement shown in FIG. 5 (c) and FIG. 6.

FIG. 7 is a schematic flowchart of a method for assisting fitness according to an embodiment of this application.

Step S601: An electronic device obtains a user movement.

The electronic device may obtain a real-time image of the user movement. In other words, the electronic device may obtain a user movement video. In the user movement video, a user is performing the user movement. The user movement video may be a video formed from an image collected by the electronic device in real time.

The electronic device may recognize joints in the user movement, to determine a first body part and a second body part in the user movement.

The electronic device may recognize skeleton joints of the user in the user movement. Skeleton joints may also be referred to as joints. A skeleton joint of the user may represent a body part of the user. The electronic device may determine a location, a movement, and the like of a body part of the user based on a location change of a skeleton joint of the user. Alternatively, a user body part may be recognized in another manner. The body parts may also be understood as body parts.

The electronic device may recognize the first body part and the second body part based on the joints.

Step S602: The electronic device determines a motion track of the first body part in the user movement.

The first body part may be one or some parts of the body.

The motion track of the first body part may refer to a spatial feature of a movement including a route through which the first body part passes from a start location to an end location. The motion track may include a motion track direction, a motion track shape, and the like. The motion track direction of the first body part may be a motion direction formed when the first body part performs the user movement. The motion track shape may be a line, a curve, or a combination thereof.

The electronic device may determine a candidate movement from the user movement based on the motion track of the first body part. The electronic device determines whether the motion track of the first body part meets a first preset condition, and uses, as the candidate movement, a user movement corresponding to a motion track that meets the first preset condition. The candidate movement is a user movement in which the motion track of the first body part meets the first preset condition.

The first preset condition may also be understood as a preset location change feature of the first body part. In the user movement, the electronic device may determine, as the candidate movement, a user movement in which the motion track of the first body part of the user meets the preset location change feature. That is, the electronic device may determine, from the user movement, the candidate movement that meets a preset feature of the motion track of the first body part. The electronic device may determine the candidate movement from the user movement video based on a feature of the motion track of the first body part.

The preset location change feature may include one or more of a shape of the motion track, a cyclicality of the motion track, and the like.

The electronic device may select, as the candidate movement, a video in which the motion track of the first body part meets the preset feature. That is, the candidate movement is a video in which the motion track has a specific feature.

For example, the electronic device may determine the candidate movement from the user movement based on the cyclicality of the motion track of the first body part. Alternatively, the electronic device may select, as the candidate movement, a user movement in which similarity between a motion track of the first body part and a preset track is less than a preset value.

The candidate movement may be a video corresponding to a cycle of the motion track of the first body part, or may be a video or an image corresponding to a segment in a cycle of the motion track of the first body part, for example, a video or an image corresponding to a track in a specific location range of the first body part in a cycle. A selection manner of the candidate movement may be determined based on a specific fitness movement.

The preset feature may be used to indicate a location change manner of a skeleton joint corresponding to the first body part. A location change manner of the first body part may be a motion direction change of the first body part.

The motion direction change of the first body part may be, for example, an upward motion, a downward motion, or an up-and-down reciprocation motion. A motion direction of the first body part may also be understood as a motion direction of all or some skeleton joints on the first body part. A first key joint is a skeleton joint on the first body part. A location change manner of the first key joint may alternatively be a shape of a motion track. For example, the motion track is in a shape such as a triangle, a circle, an arc, or a broken line.

An angle change of the first body part is an angle change of a movement of the first body part and a direction change and a relative location change between joints on the first body part.

The motion track of the first body part may be understood as a motion track of the first key joint on the first body part. The determined candidate movement may be, for example, a user movement corresponding to an upward motion of the first key joint in an up-and-down reciprocation motion process. Alternatively, when the motion track of the first key joint is a triangle, the determined candidate movement is a corresponding user movement when the first key joint moves on an edge of the triangle.

Step S603: The electronic device determines a movement change amplitude of the second body part in the candidate movement.

The electronic device may determine the movement change amplitude of the second body part in the candidate movement.

The movement change amplitude of the second body part may also be understood as a location change amplitude of the second body part, that is, a difference between two end points of a largest location change range of the second body part. The movement change amplitude may include a change angle, a change distance, and the like. In other words, the movement change amplitude of the second body part may be a difference between a largest value and a smallest value of an included angle between the second body part and the horizontal direction or the vertical direction. Alternatively, the movement change amplitude of the second body part may be a longest distance between locations that the second body part passes through in a location change process of the second body part. Alternatively, the movement change amplitude of the second body part may be a largest value of a distance change amount between second body parts.

The electronic device may determine that the movement change of the second body part in the user movement meets a largest value requirement and a smallest value requirement of a preset movement change range, to determine that the user movement is a fitness movement.

The second body part may include one or more body parts. The second body part may include all or a part of the first body part. Alternatively, the second body part may be a body part other than the first body part of the user.

The electronic device may further determine that the user movement meets the preset movement change range of the second body part, to determine that the user movement is a fitness movement.

A movement change range of the second body part in the candidate movement may include the movement change amplitude of the second body part in the candidate movement. The movement change range of the second body part in the candidate movement may include at least one of a movement start location of the second body part in the candidate movement and a movement end location of the second body part in the candidate movement.

The movement start location and the movement end location of the second body part in the candidate movement may be determined based on the motion track of the first body part. For example, the movement start location of the second body part in the candidate movement may be a location of the first body part in the first frame of image of the candidate movement. The movement end location of the second body part in the candidate movement is a location of the first body part in the first frame of image of the candidate movement. Alternatively, it may be determined that a location of the second body part in an image that is in the candidate movement and that corresponds to a point on the motion track of the first body part is the movement start location or the movement end location of the second body part.

The electronic device may store a correspondence between a location of the first body part on the motion track of the first body part and the movement start location or the movement end location of the second body part.

The location change range of the second body part in the candidate movement may be a range corresponding to the location change amplitude of the second body part in the candidate movement. The candidate movement is selected, so that the location change amplitude of the second body part is a difference corresponding to the movement start location and the movement end location of the second body part in the candidate movement. Alternatively, the location change range of the second body part in the candidate movement is used to indicate a change of a location of the second body part in the last frame of image of the candidate movement relative to a location of the second body part in the first frame of image of the candidate movement.

The largest value requirement of the preset movement change range is an interval range of a largest value of the preset movement change range, and the smallest value requirement of the preset movement change range is an interval range of a smallest value of the preset movement change range. When a largest value of the movement change range of the second body part falls within the largest value interval of the preset movement change range, and a smallest value of the movement change range of the second body part falls within the smallest value interval of the preset movement change range, it may be considered that the user movement is a fitness movement. That is, the user performs the fitness movement.

The second body part may include one or more body parts. The electronic device may determine, based on the location change range of the second body part, whether the user performs the fitness movement. The electronic device may obtain the location change range of the second body part based on the user movement. The electronic device determines, based on a stored recognition condition corresponding to the fitness movement, whether the user performs the fitness movement.

The recognition condition may be the location change range of the second body part, or the like.

A movement change range may include a location change manner. The location change manner may be, for example, an up-and-down motion, a horizontal motion, a circular motion, or an angle change. The movement change range of the second body part may include an angle change range of the second body part, or may include a change range of relative locations of the second body part.

The electronic device may recognize skeleton joints in the user movement, to determine the second body part in the user movement. The electronic device may determine that the second body part in the user movement meets the preset movement change range of the second body part.

The electronic device may determine, based on the location change range of the second body part, a type of the fitness movement performed by the user. Based on a recognition condition met by the location change of the second body part, a type of a fitness movement corresponding to the recognition condition may be determined.

The electronic device may obtain input information before step S601 or before step S602. The input information is used to indicate the fitness movement. The electronic device determines, based on the input information, the first preset condition, namely, the preset condition that the motion track of the first body part meets.

The electronic device may determine at least one of the first body part, the second body part, a third body part, a fourth body part, and the like corresponding to the fitness movement.

The electronic device may determine the fitness movement based on the input information. In other words, the electronic device may determine the type of the fitness exercise based on the input information. For different fitness movements, the second body part may be different body parts. The recognition condition may be the location change range of the second body part. Therefore, the recognition conditions may be different for different fitness movements. The electronic device may determine, based on a recognition condition corresponding to this type of fitness movement, whether the user performs the fitness movement.

The recognition condition may include the movement change amplitude of the second body part, or the recognition condition may include at least one of the movement start location of the second body part and the movement end location of the second body part. When the second body part in the user movement meets the recognition condition, it may be determined that the user movement is the fitness movement, that is, the user is performing the fitness movement.

The input information indicates the fitness movement, so that the electronic device can determine, based on only the range of the second body part corresponding to the fitness movement, whether the user performs the fitness movement, and does not need to determine whether the user movement meets another recognition condition of the fitness movement, to reduce a calculation amount. The movement change range of the second body part may include the movement change amplitude of the second body part, or may include the movement start location of the second body part and the movement end location of the second body part.

The movement start location of the second body part and the movement end location of the second body part may be determined based on the motion track of the first body part. The movement start location of the second body part may be the movement start location of the second body part in the candidate movement, namely, a location of the second body part in the first frame of image corresponding to the candidate movement. Alternatively, the movement start location of the second body part may be a location of the second body part in an image that is of the candidate movement and that corresponds to a point on which the first body part is located on the motion track.

The location change range of the second body part of the user may be a location change range of the second body part in the entire user movement, that is, may be a range corresponding to a largest value of the location change amplitude of the second body part in the user movement. The electronic device may select any two frames of images each time in the user movement, compare locations of the second body part, and determine the largest value of the location change amplitude of the second body part by selecting frames of images for a plurality of times and comparing locations of the second body part.

Alternatively, the location change range of the second body part of the user may be a location change range in the candidate movement of the user movement. The candidate movement is a video in the user movement.

Before step S602, the electronic device may determine whether the user movement includes the first body part of the user.

When the user movement includes the first body part of the user, step S602 is performed.

When the user movement does not include the first body part, the electronic device may output prompt information. The prompt information may be used to prompt the user to adjust an image collection range of the user movement. In this case, the electronic device may not perform subsequent steps.

The electronic device may further determine whether the user movement includes the second body part of the user. When the user movement does not include the second body part, the electronic device may output prompt information. The prompt information may be used to prompt the user to adjust an image collection range of the user movement.

Before step S602, the electronic device may further determine a horizontal direction or a vertical direction of the user movement. The electronic device may determine a location of a body part of the user, a change of the location, a motion track of the body part, and the like based on the horizontal direction or the vertical direction.

Step S604: The electronic device determines, based on the movement change amplitude, to output guidance information.

When the user performs the fitness movement, the electronic device may provide an evaluation and guidance for the user movement.

The electronic device may determine, based on whether the movement change amplitude of the second body part meets a second preset condition, whether to output the guidance information. The second preset condition may also be referred to as a recognition condition.

When determining that the movement change amplitude meets the second preset condition, the electronic device determines to output the guidance information. When the movement change amplitude of the second body part meets the second preset condition, it is considered that the user performs the fitness movement.

In some embodiments, the electronic device may determine a similarity between the user movement and a standard movement of the fitness movement, and provide an evaluation and guidance for the user movement.

In some other embodiments, for the fitness movement, the electronic device may store a correspondence between a user movement and evaluation information. The electronic device may indicate a user movement by using location information of a body part of the user. The electronic device may store a correspondence between location information of the first body part of the user and evaluation information.

The location information of the second body part may be used to indicate a location of the second body part, and the location of the second body part may be a location change range or a location at a specific time point.

The location information of the second body part includes at least one of the movement change amplitude of the second body part, the movement start location of the second body part, the movement end location of the second body part, and a motion track of the second body part.

The electronic device may determine, based on the motion track of the first body part, a time point on which the second body part is located at a location, determine a user movement on the time point, and determine location information of the second body part on the time point, so as to determine corresponding evaluation information based on the correspondence between location information of the second body part and evaluation information.

The electronic device may determine the movement start location of the second body part and the movement end location of the second body part based on a stored correspondence between a location of the first body part in the motion track of the first body part and each of the movement start location of the second body part and the movement end location of the second body part. The electronic device determines, based on whether the movement start location of the second body part and the movement end location of the second body part meet a recognition condition, whether to output the guidance information.

The electronic device may determine, based on the motion track of the first body part, a period of time in which the first body part is located in a range, and determine location information of the second body part in the period of time, to determine corresponding evaluation information based on the correspondence between location information of the second body part and evaluation information.

The electronic device may determine the corresponding evaluation information based on a location change status of the first body part.

For example, the location information of the second body part may be a largest value or a smallest value of an included angle between the second body part and the horizontal or vertical direction in the user movement or the candidate movement, or may be a change range of the included angle, that is, a range from a smallest value to a largest value or a range from a smallest value to a largest value of the included angle. The location of the second body part may alternatively be a ratio of a motion distance of the second body part to a length of the second body part. The location of the second body part may alternatively be a relative location relationship between the second body part and another body part, a relative relationship between second body parts, or the like.

When the user performs the fitness movement, the electronic device determines first evaluation information corresponding to first location information. The first location information is used to indicate a location of the second body part in the user movement.

The location information of the second body part may one-to-one correspond to the evaluation information. The location information of the second body part may include at least one of the movement change range of the second body part, the movement change range amplitude of the second body part, a limit value of the movement change range of the second body part, and the motion track of the second body part.

The limit value of the movement change range of the second body part is a largest value or a smallest value of the movement change range of the second body part. For example, the value may include a largest value or a smallest value of an angle of the second body part, or may include a largest value or a smallest value of a distance between second body parts, that is, a largest value or a smallest value of a distance between a body part and another body part.

The movement change amplitude of the second body part may also be referred to as the movement change range amplitude, that is, a difference between the largest value and the smallest value of the movement change range of the second body part.

The correspondence between the motion track of the second body part and the evaluation information may be, for example, a correspondence between a shape, a cycle, and the like of the motion track of the second body part and the evaluation information.

The electronic device may output the guidance information based on the first evaluation information. The first evaluation information may include a score and/or an evaluation. The guidance information may be the same as or different from the first evaluation information. The electronic device may adjust the first evaluation information based on a movement completion status of another body part of the user, to obtain the guidance information.

The electronic device may determine second evaluation information corresponding to second location information of the user. The second location information is used to indicate a location of the third body part in the user movement. The second evaluation information may be, for example, a score and/or an evaluation. The second location information may include one or more of a movement change range of the third body part, a movement change amplitude of the third body part, a limit value of the movement change range of the third body part, and a motion track of the third body part.

The electronic device may output the guidance information based on the second evaluation information and the first evaluation information.

When a score corresponding to the location of the second body part is greater than a preset value, the guidance information may include the evaluation in the second evaluation information. When a score that is in the first evaluation information and that corresponds to the location of the second body part is greater than or equal to the preset value, the electronic device may output the evaluation in the second evaluation information. Conversely, when the score that is in the first evaluation information and that corresponds to the location of the second body part is less than the preset value, the guidance information may include only the evaluation in the first evaluation information.

For the fitness movement, when a movement completed by a main body part of the user is not standard, guidance may be provided on only the movement of the main body part, and when the movement completed by the main body part of the user is relatively standard, guidance may be provided on a movement of another body part. When the score is less than the preset value, the electronic device may not determine the second evaluation information.

The electronic device may adjust the score in the first evaluation information based on the score in the second evaluation information, to determine a score in the guidance information.

The score in the second evaluation information may be a score by which the score in the first evaluation information is increased or decreased, and the score in the first evaluation information may be increased or decreased by the score. Alternatively, the score in the second evaluation information may be a score of a movement of the third body part. The electronic device may increase or decrease, based on a weight of the third body part, the score in the first evaluation information by a value obtained by multiplying the score in the second evaluation information by the weight.

The third body part may include one or more body parts. The third body part may be a body part other than the second body part of the user.

The electronic device may determine whether the user movement includes the third body part of the user. When the user movement does not include the third body part, the electronic device may output prompt information. The prompt information may be used to prompt the user to adjust an image collection range of the user movement.

After steps S601 and S602, the electronic device can accurately determine whether the user performs the fitness movement, so that guidance can be provided when the user performs the fitness movement, to improve user experience.

FIG. 9 is a schematic diagram of a structure of an electronic apparatus according to an embodiment of this application. An apparatus 700 includes an obtaining module 701 and a determining module 702.

The obtaining module 701 is configured to obtain a user movement.

The determining module 702 is configured to determine, from the user movement, a candidate movement in which a motion track of a first body part in the user movement meets a first preset condition.

The determining module 702 is further configured to determine a movement change amplitude of a second body part in the candidate movement.

The determining module 702 is further configured to determine, based on the movement change amplitude, to output guidance information.

Optionally, the apparatus 700 further includes a judgment module, configured to determine that the movement change amplitude meets a second preset condition.

The determining module 702 is further configured to determine to output the guidance information.

Optionally, the obtaining module 701 is further configured to obtain input information.

The determining module 702 is further configured to determine the first preset condition corresponding to the input information.

Optionally, the determining module 702 is further configured to determine first evaluation information corresponding to first location information, where the first location information includes at least one of a movement change amplitude of the second body part, a movement start location of the second body part, a movement end location of the second body part, and a motion track of the second body part, and the movement start location of the second body part and the movement end location of the second body part are determined based on the motion track of the first body part.

The apparatus 700 further includes an output module, configured to output the guidance information based on the first evaluation information.

Optionally, the determining module 702 is further configured to determine second evaluation information corresponding to second location information of the user, where the second location information includes at least one of a movement change amplitude of a third body part, a movement start location of the third body part, a movement end location of the third body part, and a motion track of the third body part, and the movement start location of the third body part and the movement end location of the third body part are determined based on the motion track of the first body part.

The output module is further configured to output the guidance information based on the second evaluation information and the first evaluation information.

Optionally, the apparatus 700 further includes a recognition module, configured to recognize joints in the user movement, to determine the first body part and the second body part in the user movement.

Optionally, the determining module 702 is further configured to determine, based on the movement change amplitude and the movement start location of the second body part in the candidate movement, to output the guidance information; or determine, based on the movement change amplitude and the movement end location of the second body part in the candidate movement, to output the guidance information; or determine, based on the movement change amplitude, and the movement start location and the movement end location of the second body part in the candidate movement, to output the guidance information, where the movement start location of the second body part and the movement end location of the second body part are determined based on the motion track of the first body part.

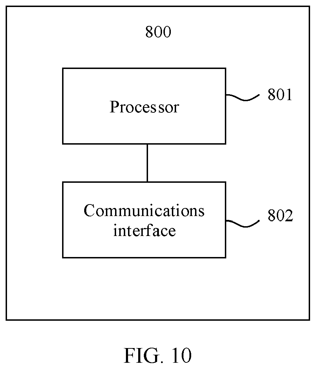

FIG. 10 is a schematic diagram of a structure of an electronic apparatus according to an embodiment of this application. An apparatus 800 includes a processor 801 and a communications interface 802.

The communications interface 802 is configured to obtain a user movement.

The processor 801 is configured to determine, from the user movement, a candidate movement in which a motion track of a first body part in the user movement meets a first preset condition; determine a movement change amplitude of a second body part in the candidate movement; and determine, based on the movement change amplitude, to output guidance information.

Optionally, the processor 801 is configured to determine that the movement change amplitude meets a second preset condition, and determine to output the guidance information.

Optionally, the communications interface 802 is further configured to obtain input information.

The processor 801 is further configured to determine the first preset condition corresponding to the input information.

Optionally, the processor 801 is configured to determine first evaluation information corresponding to first location information, where the first location information includes at least one of a movement change amplitude of the second body part, a movement start location of the second body part, a movement end location of the second body part, and a motion track of the second body part, and the movement start location of the second body part and the movement end location of the second body part are determined based on the motion track of the first body part; and determine the guidance information based on the first evaluation information.

Optionally, the processor 801 is configured to determine second evaluation information corresponding to second location information of the user, where the second location information includes at least one of a movement change amplitude of a third body part, a movement start location of the third body part, a movement end location of the third body part, and a motion track of the third body part, and the movement start location of the third body part and the movement end location of the third body part are determined based on the motion track of the first body part; and determine the guidance information based on the second evaluation information and the first evaluation information.

Optionally, the processor 801 is further configured to recognize joints in the user movement, to determine the first body part and the second body part in the user movement.

Optionally, the processor 801 is further configured to determine, based on the movement change amplitude and the movement start location of the second body part in the candidate movement, to output the guidance information; or determine, based on the movement change amplitude and the movement end location of the second body part in the candidate movement, to output the guidance information; or determine, based on the movement change amplitude, and the movement start location and the movement end location of the second body part in the candidate movement, to output the guidance information, where the movement start location of the second body part and the movement end location of the second body part are determined based on the motion track of the first body part.

The embodiments of this application further provide an electronic apparatus, including at least one processor and a communications interface. The communications interface is used by the electronic apparatus to exchange information with another electronic apparatus, and when program instructions are executed by the at least one processor, the electronic apparatus is enabled to perform the foregoing method.

The embodiments of this application further provide a computer program storage medium. The computer program storage medium includes program instructions. When the program instructions are directly or indirectly executed, the method in the foregoing descriptions is implemented.

The embodiments of this application further provide a chip system. The chip system includes at least one processor, and when program instructions are executed by the at least one processor, the method in the foregoing descriptions is performed.

A person of ordinary skill in the art may be aware that, in combination with the examples described in the embodiments disclosed in this specification, units and algorithm steps may be implemented by electronic hardware or a combination of computer software and electronic hardware. Whether the functions are performed by hardware or software depends on particular applications and design constraint conditions of the technical solutions. A person skilled in the art may use different methods to implement the described functions for each particular application, but it should not be considered that the implementation goes beyond the scope of this application.

A person skilled in the art may clearly understand that, for the purpose of convenient and brief description, for detailed working processes of the foregoing system, apparatus, and units, refer to corresponding processes in the foregoing method embodiment. Details are not described herein again.

In description of the embodiments of this application, "/" means "or" unless otherwise specified. For example, A/B may represent A or B. In this specification, "and/or" describes only an association relationship of associated objects and represents that three relationships may exist. For example, A and/or B may represent the following three cases: Only A exists, both A and B exist, and only B exists. In addition, in the descriptions of the embodiments of this application, unless otherwise specified, "a plurality of' means two or more.

Terms "first" and "second" are merely used for description, but shall not be construed as an indication or implication of relative importance or as an implication of a quantity of technical features. Therefore, a feature limited by "first" or "second" may explicitly or implicitly include one or more features.

In the several embodiments provided in this application, it should be understood that the disclosed system, apparatus, and method may be implemented in other manners. For example, the described apparatus embodiment is merely an example. For example, division into the units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one location, or may be distributed on a plurality of network units. Some or all of the units may be selected based on actual requirements to achieve the objectives of the solutions of the embodiments.

In addition, function units in the embodiments of this application may be integrated into one processing unit, or each of the units may exist alone physically, or two or more units are integrated into one unit.

When the functions are implemented in a form of a software function unit and sold or used as an independent product, the functions may be stored in a computer readable storage medium. Based on such an understanding, the technical solutions of this application essentially, or the part contributing to the conventional technology, or some of the technical solutions may be implemented in a form of a software product. The computer software product is stored in a storage medium, and includes several instructions for instructing a computer device (which may be a personal computer, a server, a network device, or the like) to perform all or some of the steps of the methods in the embodiments of this application. The foregoing storage medium includes: any medium that can store program code, such as a USB flash drive, a removable hard disk, a read-only memory (read-only memory, ROM), a random access memory (random access memory, RAM), a magnetic disk, or an optical disc.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A method for assisting fitness, comprising:
obtaining, by an electronic device, a user movement;
determining, by the electronic device from the user movement, a candidate movement in which a motion track of a first body part in the user movement meets a first preset condition;
determining, by the electronic device, a movement change amplitude of a second body part in the candidate movement; and
determining, by the electronic device based on the movement change amplitude, to output guidance information.

2. The method according to claim 1, wherein the determining, by the electronic device based on the movement change amplitude, to output guidance information comprises:
determining, by the electronic device, that the movement change amplitude meets a second preset condition; and
determining, by the electronic device, to output the guidance information.

3. The method according to claim 1 or 2, wherein the method further comprises:
obtaining, by the electronic device, input information; and
determining, by the electronic device, the first preset condition corresponding to the input information.

4. The method according to any one of claims 1 to 3, wherein the method further comprises:
determining, by the electronic device, first evaluation information corresponding to first location information, wherein the first location information comprises at least one of the movement change amplitude of the second body part, a movement start location of the second body part, a movement end location of the second body part, and a motion track of the second body part, and the movement start location of the second body part and the movement end location of the second body part are determined based on the motion track of the first body part; and
outputting, by the electronic device, the guidance information based on the first evaluation information.

5. The method according to claim 4, wherein the method further comprises: determining, by the electronic device, second evaluation information corresponding to second location information of a user, wherein the second location information comprises at least one of a movement change amplitude of a third body part, a movement start location of the third body part, a movement end location of the third body part, and a motion track of the third body part, and the movement start location of the third body part and the movement end location of the third body part are determined based on the motion track of the first body part; and
the outputting, by the electronic device, the guidance information based on the first evaluation information comprises: outputting, by the electronic device, the guidance information based on the second evaluation information and the first evaluation information.

6. The method according to any one of claims 1 to 5, wherein the method further comprises: recognizing joints in the user movement, to determine the first body part and the second body part in the user movement.

7. The method according to any one of claims 1 to 6, wherein the determining, by the electronic device based on the movement change amplitude, to output guidance information comprises:
determining, by the electronic device based on the movement change amplitude and the movement start location of the second body part in the candidate movement, to output the guidance information; or
determining, by the electronic device based on the movement change amplitude and the movement end location of the second body part in the candidate movement, to output the guidance information; or
determining, by the electronic device based on the movement change amplitude, and the movement start location and the movement end location of the second body part in the candidate movement, to output the guidance information, wherein
the movement start location of the second body part and the movement end location of the second body part are determined based on the motion track of the first body part.

8. An electronic apparatus, comprising a processor and a communications interface, wherein
the communications interface is configured to obtain a user movement; and
the processor is configured to:
determine, from the user movement, a candidate movement in which a motion track of a first body part in the user movement meets a first preset condition;
determine a movement change amplitude of a second body part in the candidate movement; and
determine, based on the movement change amplitude, to output guidance information.

9. The apparatus according to claim 8, wherein the processor is configured to:
determine that the movement change amplitude meets a second preset condition; and
determine to output the guidance information.

10. The apparatus according to claim 8 or 9, wherein
the communications interface is further configured to obtain input information; and
the processor is further configured to determine the first preset condition based on the input information.

11. The apparatus according to any one of claims 8 to 10, wherein the processor is configured to:
determine first evaluation information corresponding to first location information, wherein the first location information comprises at least one of the movement change amplitude of the second body part, a movement start location of the second body part, a movement end location of the second body part, and a motion track of the second body part, and the movement start location of the second body part and the movement end location of the second body part are determined based on the motion track of the first body part; and
determine the guidance information based on the first evaluation information.

12. The apparatus according to claim 11, wherein the processor is configured to:
determine second evaluation information corresponding to second location information of the user, wherein the second location information comprises at least one of a movement change amplitude of a third body part, a movement start location of the third body part, a movement end location of the third body part, and a motion track of the third body part, and the movement start location of the third body part and the movement end location of the third body part are determined based on the motion track of the first body part; and
determine the guidance information based on the second evaluation information and the first evaluation information.

13. The apparatus according to any one of claims 8 to 12, wherein the processor is further configured to recognize joints in the user movement, to determine the first body part and the second body part in the user movement.

14. The apparatus according to any one of claims 8 to 13, wherein the processor is further configured to:
determine, based on the movement change amplitude and the movement start location of the second body part in the candidate movement, to output the guidance information; or
determine, based on the movement change amplitude and the movement end location of the second body part in the candidate movement, to output the guidance information; or
determine, based on the movement change amplitude, and the movement start location and the movement end location of the second body part in the candidate movement, to output the guidance information, wherein
the movement start location of the second body part and the movement end location of the second body part are determined based on the motion track of the first body part.

15. A computer storage medium, wherein when the computer instructions are run on an electronic device, the electronic device is enabled to perform the method according to any one of claims 1 to 7.

16. A chip system, wherein the chip system comprises at least one processor, and when program instructions are executed in the at least one processor, the chip system is enabled to perform the method according to any one of claims 1 to 7.
